# EUROPEAN PATENT APPLICATION

(11) **EP 4 571 757 A1**
(43) Date of publication of application: **18.06.2025**
(21) Application number: 22955109.8
(22) Date of filing: 11.11.2022
(51) Int. Cl.: G16H 20/00, G16H 50/20, G16H 50/70, G16H 50/30, A61B 5/00, G16H 50/50, G10L 15/02

(54) **METHOD AND APPARATUS FOR IMPLEMENTING APPLICATION FOR MAINTAINING COGNITIVE FUNCTION AND IMPROVING DECLINE IN COGNITIVE FUNCTION**

(30) Priority: 09.08.2022 KR 20220099563; 08.11.2022 KR 20220148127
(71) Applicant: Emocog Inc., Seoul 08708 (KR)
(72) Inventor: NOH, Yoo Hun, Seoul 06346 (KR)
(74) Representative: Zacco Sweden AB
(86) International application number: PCT/KR2022/017736
(87) International publication number: WO 2024/034737

(57) **Abstract**

Provided is a method of implementing an application for improving mild cognitive impairment symptoms, wherein the method includes: controlling a first training algorithm to be output to a user terminal in response to an input into the user terminal; in a case where the first training algorithm is terminated, controlling a second training algorithm to be output to the user terminal; in a case where the second training algorithm is terminated, evaluating user inputs into the first training algorithm and the second training algorithm; and on the basis of a result of the evaluation, controlling a first visual display to be output to the user terminal.

## Description

### Technical Field

The present disclosure relates to a method of implementing an application, and more particularly, to a method of implementing an application for maintaining a cognitive function and improving a decline in the cognitive function, and an apparatus for the method.

### Background Art

It is evaluated that South Korea has already entered an aging society. As the aging society progresses, the number of dementia patients may rapidly increase, and social costs incurred to treat and care for dementia patients may also gradually increase.

It has been proven that training capable of stimulating areas of the brain responsible for cognitive functions is effective in preventing dementia and improving symptoms of dementia that has already developed, and it has been proven through several papers that, in the case where patients with mild cognitive impairment corresponding to a pre-dementia stage receive training capable of improving cognitive functions, cognitive functions of the patients improve to a greater extent than in patients who already have dementia.

### Disclosure

### Technical Problem

The technical problems to be solved by the present disclosure are to provide a method of implementing an application for maintaining a cognitive function and improving a decline in the cognitive function, and an apparatus for the method.

### Technical Solution

A method according to an embodiment of the present disclosure for solving the technical problems includes: controlling a first training algorithm to be output to a user terminal in response to an input into the user terminal; in a case where the first training algorithm is terminated, controlling a second training algorithm to be output to the user terminal; in a case where the second training algorithm is terminated, evaluating user inputs into the first training algorithm and the second training algorithm; and on the basis of a result of the evaluation, controlling a first visual display to be output to the user terminal.

An embodiment of the present disclosure may provide a computer program stored in a computer-readable storage device to execute the method.

An apparatus according to another embodiment of the present disclosure for solving the technical problems includes: a first output controller configured to control a first training algorithm to be output to a user terminal in response to an input into the user terminal; a second output controller configured to control a second training algorithm to be output to the user terminal in a case where the first training algorithm is terminated; a training evaluation unit configured to evaluate user inputs into the first training algorithm and the second training algorithm in a case where the second training algorithm is terminated; and a visual display controller configured to control a first visual display to be output to the user terminal on the basis of a result of the evaluation.

### Advantageous Effects

According to the present disclosure, symptoms of a user suffering from cognitive impairment may be significantly improved.

### Description of Drawing

FIG. 1 is a view schematically illustrating an entire system for implementing the present disclosure.
FIG. 2 is a block diagram illustrating, as a logical device, a training app according to an embodiment of the present disclosure.
FIG. 3 is a block diagram illustrating an example of a processing unit subdivided by function.
FIG. 4 is a flowchart illustrating an example of an operation process of submodules of the processing unit of FIG. 3.
FIG. 5 is a flowchart illustrating an example of a process of a first training algorithm and a second training algorithm.
FIG. 6 is a flowchart illustrating an example of a process in which a visual effect of a first visual display is improved.
FIG. 7 is a table illustrating an example of detailed training algorithms included in a first training algorithm.
FIG. 8 is a table illustrating a criterion for changing a difficulty level of a detailed training algorithm corresponding to a focusing functional area in a first training algorithm.
FIG. 9 is a table illustrating a criterion for changing a difficulty level of a detailed training algorithm corresponding to a visualization functional area in a first training algorithm.
FIG. 10 is a table illustrating a criterion for changing a difficulty level of a detailed training algorithm corresponding to a fusion functional area in a first training algorithm.
FIG. 11 is a table illustrating an example of detailed training algorithms included in a second training algorithm.
FIG. 12 is a table illustrating a criterion for changing a difficulty level of training "memorizing repeatedly" in a second training algorithm.
FIG. 13 is a table illustrating a criterion for changing a difficulty level of training "exchanging a word" in a second training algorithm.
FIG. 14 is a table illustrating a criterion for changing a difficulty level of training "clapping" in a second training algorithm.
FIGS. 15A and 15B are tables illustrating criteria and example sentences for changing a difficulty level of training "remembering an order" in a second training algorithm.
FIG. 16 is a table illustrating a criterion for changing a difficulty level of training "searching a word" in a second training algorithm.
FIG. 17 is a table illustrating a criterion for changing a difficulty level of training "remembering appearance" in a second training algorithm.
FIG. 18 is a table illustrating a criterion for changing a difficulty level of training "throwing a word" in a second training algorithm.
FIG. 19 is a table illustrating a criterion for changing a difficulty level of training "memorizing absurdly" in a second training algorithm.
FIG. 20 is a table illustrating a criterion for changing a difficulty level of training "making into one" in a second training algorithm.
FIG. 21 is a table schematically illustrating a warming up algorithm that may be selectively performed before a first training algorithm is performed.
FIG. 22 is a table illustrating a meditation algorithm that may be selectively performed before a second training algorithm is performed.
FIG. 23 is a view illustrating a first visual display that is output through a user terminal.
FIG. 24 is a view schematically illustrating an example of a badge that may be acquired by a user.
FIG. 25 is a view schematically illustrating another example of a badge that may be acquired by a user.
FIG. 26 is a view schematically illustrating another example of a badge that may be acquired by a user.
FIG. 27 is a view illustrating a bud of a flower of memory, which is an example of a first visual display.
FIG. 28 is a view illustrating a flower of memory in full bloom, which is another example of a first visual display.
FIG. 29 is a view illustrating a flower of gold memory in full bloom, which is another example of a first visual display.
FIG. 30 is a view illustrating archiving of a flower of memory.

### Best Mode

A method according to an embodiment of the present disclosure for solving the above technical problems includes: controlling a first training algorithm to be output to a user terminal in response to an input into the user terminal; in a case where the first training algorithm is terminated, controlling a second training algorithm to be output to the user terminal; in a case where the second training algorithm is terminated, evaluating user inputs into the first training algorithm and the second training algorithm; and on the basis of a result of the evaluation, controlling a first visual display to be output to the user terminal.

The input into the user terminal may be login information of a user input through an input device of the user terminal.

The login information may be information authenticated by a prescription code of a preset number of digits input while the login information is generated.

The controlling the second training algorithm to be output to the user terminal may include controlling the second training algorithm to be output after a certain time elapses after the first training algorithm is terminated.

The certain time may be one hour.

The first training algorithm may include a fixed first number of detailed training algorithms.

The second training algorithm may include a third number of detailed training algorithms selected from a fixed second number of detailed training algorithms according to a preset condition.

A detailed training algorithm included in the second training algorithm may be newly determined each time the user logs in.

The detailed training algorithm included in the second training algorithm may be determined on the basis of a result of evaluation of an input from the user to the first training algorithm.

The second number may be nine, and the third number may be four.

The first training algorithm may include a detailed training algorithm for a focusing functional area.

The detailed training algorithm for the focusing functional area may be an algorithm configured to receive and evaluate speech of the user using the user terminal.

The detailed training algorithm for the focusing functional areas may include: providing a guidance message to the user through the user terminal; and receiving the speech of the user having at least one of a pitch and a speed changed according to the guidance message, comparing the user speech with a stored value, and evaluating the user speech.

The first training algorithm may include a detailed training algorithm for a visualization functional area.

The detailed training algorithm for the visualization functional area may include an algorithm configured to receive the speech of the user using the user terminal and evaluate a number of syllables of a word included in the speech.

The detailed training algorithm for the visualization functional area may include: providing a guidance message to the user through the user terminal; and receiving the speech of the user for a certain period of time in response to the guidance message, detecting a number of syllables, comparing the detected number of syllables with a preset value, and evaluating the speech of the user.

The first training algorithm may include a detailed training algorithm for a fusion functional area.

The detailed training algorithm for the fusion functional area may include an algorithm configured to receive a story created by the user using the user terminal and determine whether or not the story meets a condition.

The detailed training algorithm for the fusion functional area may include: providing a story creation condition to the user through the user terminal; and in a case where the story created by the user is received, determining whether or not the story is a story corresponding to a number of words and emotional modifiers included in the story creation condition.

The first training algorithm may include at least two of detailed training algorithms for focusing, visualization, and fusion functional areas.

The method may further include controlling a warming up algorithm configured to give a certain stimulation to a cognitive functional area of the user to be output by guiding the user to physically move before controlling the first training algorithm to be output to the user terminal, wherein evaluation of the warming up algorithm may be excluded from a result of the evaluation.

The first training algorithm may include at least two detailed training algorithms, and a difficulty level may be set for each of the detailed training algorithms.

When the detailed training algorithm is executed at a lowest difficulty level, a guide message for the detailed training algorithm may be provided through the user terminal.

The guide message may be audibly provided.

In a case where a history in which a difficulty level is higher than the lowest difficulty level and then lowered again is detected, the guide message may be controlled to be omitted.

A change in the difficulty level may be set to increase or decrease in real time while the detailed training algorithm is provided, according to an average correct answer rate of recent answers to the first training algorithm performed in a question-and-answer format.

Before any one of the first training algorithm and the second training algorithm is executed, a login history may be detected and a reward may be provided on the basis of the detected login history.

An accumulated number of logins may be calculated, and a first reward may be provided according to the calculated accumulated number of logins.

The first reward may be controlled to be output as a second visual display by shaping water through the user terminal.

The controlling the first visual display to be output may include, in a case where the second visual display is output, changing a visual effect of the first visual display on the basis of history information in which the second visual display is output.

A number of consecutive logins in which the logins are continuously performed may be calculated, and a second reward may be provided according to the calculated number of consecutive logins.

The second reward may be controlled to be output as a third visual display by shaping at least one of sunlight and sunshine through the user terminal.

The controlling the first visual display to be output may include, in a case where the third visual display is output, changing a visual effect of the first visual display on the basis of history information in which the third visual display is output.

The calculating the accumulated number of logins and the number of consecutive logins in which the logins are continuously performed, the providing at least one of a first reward and a second reward according to the calculated accumulated number and the number of consecutive logins, the controlling the first reward to be output as a second visual display by shaping water through the user terminal, the controlling the second reward to be output as a third visual display by shaping at least one of sunlight and sunshine through the user terminal, and the controlling the first visual display to be output may include, in a case where the second visual display and the third visual display are output, changing the visual effect of the first visual display on the basis of the history information in which the third visual display is output and, in a case where results of evaluation of the first training algorithm and the second training algorithm exceed a threshold value, controlling a fourth visual display to be output.

In a case where the fourth visual display is output, the visual effect of the first visual display may be changed on the basis of history information in which the fourth visual display is output.

The first visual display may be output in a first form after the first training algorithm is terminated and may be output in a second form improved from the first form after the second training algorithm is terminated.

The first form may be a shape obtained by shaping a flower bud, and the second form may be a shape obtained by shaping the flower bud in full bloom.

The controlling the second training algorithm to be output to the user terminal may include controlling a meditation algorithm corresponding to the second training algorithm to be output before the second training algorithm is executed.

The first visual display may have a form obtained by shaping a flowerpot, a stem grown in the flowerpot, and a flower blooming from the stem.

An embodiment of the present disclosure may provide a computer program stored in a computer-readable storage device to execute the method.

An apparatus according to another embodiment of the present disclosure for solving the above technical problems includes: a first output controller configured to control a first training algorithm to be output to a user terminal in response to an input into the user terminal; a second output controller configured to control a second training algorithm to be output to the user terminal in a case where the first training algorithm is terminated; a training evaluation unit configured to evaluate user inputs into the first training algorithm and the second training algorithm in a case where the second training algorithm is terminated; and a visual display controller configured to control a first visual display to be output to the user terminal on the basis of a result of the evaluation.

### Mode for Invention

The present disclosure may be modified in various ways and have various embodiments, and particular embodiments are illustrated in the drawings and described in detail through the detailed description. The effects and features of the present disclosure and the method for achieving them will become clear with reference to the embodiments described in detail below together with the drawings. However, the present disclosure is not limited to the embodiments provided below and may be implemented in various forms.

Hereinafter, embodiments of the present disclosure will be described in detail with reference to the attached drawings, and when describing with reference to the drawings, the same or corresponding components are given the same drawing reference numerals and the same descriptions thereof will be omitted.

In the following embodiments, the terms first, second, etc. are used for the purpose of distinguishing one component from another component, not in a limited sense.

In the following embodiments, the singular expression includes the plural expression unless the context clearly indicates otherwise.

In the embodiments below, the terms such as include or have refer to the presence of features or components described herein and do not preclude the possibility of one or more other features or components being added in advance.

In the case where an embodiment may be implemented differently, particular process sequences may also be performed differently from the order described. For example, two processes described in succession may be performed substantially simultaneously, or may be performed in the opposite order to the described order.

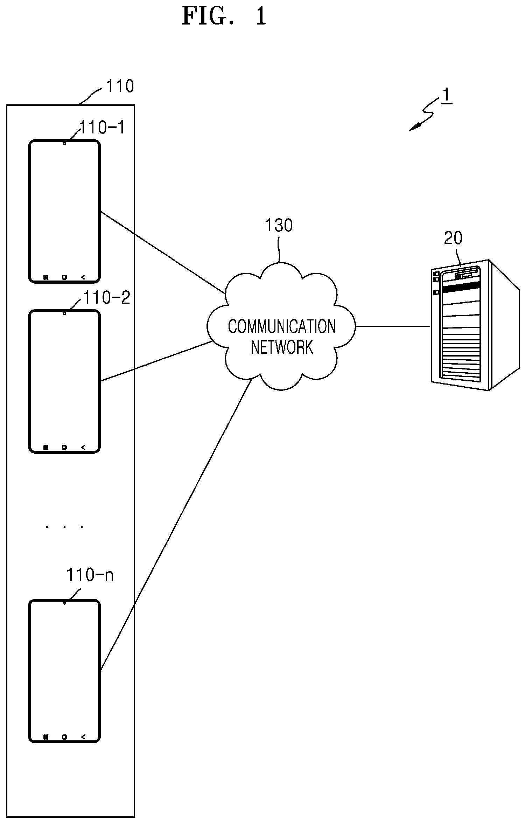

FIG. 1 is a view schematically illustrating an entire system for implementing the present disclosure.

Referring to FIG. 1, an entire system 1 according to the present disclosure has a structure in which a user terminal group 110 and a management server 20 are connected through a communication network 130.

The user terminal group 110 may include at least one user terminal. For example, the user terminal group 110 may include one user terminal, or may include n user terminals as illustrated in FIG. 1. In FIG. 1, n may be an integer equal to 1 or greater than 1.

Respective user terminals included in the user terminal group 110 are terminals of users who use a training program according to the present disclosure, and refer to electronic devices mounted with communication modules capable of communicating with the management server 20.

A user terminal refers to a smart device including an input device that receives an input from a user, an output device (a display) that visually outputs an input into the user terminal or the result of processing by the user terminal, and a communication module capable of communicating with an external device and thus is not limited to the size or type in the case where the user terminal includes the input device, the output device, and the communication module described above. For example, in FIG. 1, the user terminal is illustrated in the form of a smartphone, but the user terminal may be a PC, netbook, a netbook, or the like that may communicate with the management server 20.

A user using the user terminal refers to a person who uses an application for improving symptoms of a patient with cognitive impairment, and may be a patient who is diagnosed with cognitive impairment or a guardian of the patient. As another example, the user may be a tester who repeatedly executes the application to improve performance thereof.

The management server 20 is a server in which an integrated management program is installed, and refers to a server that manages and controls a data flow while communicating with a plurality of user terminals included in the user terminal group 110. The integrated management program (an integrated management app) is installed in the management server 20, and according to an embodiment, a portion of the integrated management program may be implemented in the form of a client driven in a user terminal and installed in the user terminals included in the user terminal group 110.

The communication network 130 performs a function of connecting a user terminal included in the user terminal group 110 to the management server 20, and may include various types of wired and wireless communication networks such as a data network, a mobile communication network, and the Internet.

In the present disclosure, an application refers to a logical device capable of controlling a screen, which is output from a user terminal included in the user terminal group 110, under control of the management server 20. The application does not have a physical form and may go through a process, which becomes optimized for a user who uses the application, several times. In other words, the application may be continuously updated on the basis of an input that is input by the user through the user terminal and a command received from the management server 20. Patients with cognitive impairment may improve cognitive impairment symptoms by repeatedly using applications running on user terminals. The application is a program implemented as a process that may improve cognitive impairment symptoms and thus is abbreviated as a "training app" hereinafter for convenience of description.

The training app may be installed and driven on at least one of a user terminal and the management server 20. As an example, the training app may be installed in a user terminal and control various types of training algorithms, which may improve cognitive impairment symptoms of a user, to be output visually, auditory, and tactilely. As another example, the training app may be implemented in a form that is installed on the management server 20 and controls contents displayed on a display of a user terminal. In this case, only a simple client that visually, auditory, and tactilely displays data received from the management server 20 is installed on the user terminal, and a function of flexibly controlling execution of a training algorithm according to an input from a user input is performed by the training app installed on the management server 20. As another example, the training app may also be implemented in a form in which the training app is separately installed on the user terminal and the management server 20. In other words, in the case where only operation characteristics of the training app fall within the scope of the present disclosure, the scope of the present disclosure is not limited by a form in which the training app is installed or a type of a device in which the training app is installed.

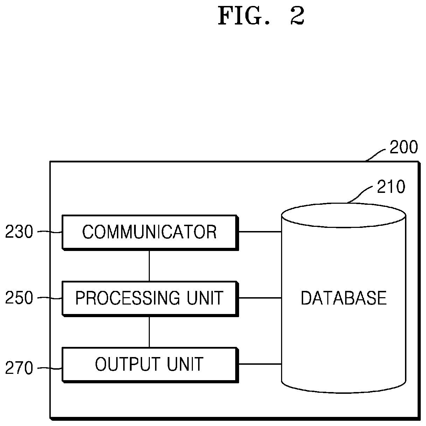

FIG. 2 is a block diagram illustrating, as a logical device, a training app according to an embodiment of the present disclosure.

A training app is not physically detected, but is a device that is logically present to implement a function for improving cognitive impairment symptoms, according to the present disclosure, and hereinafter, the training app may be referred to as a "training device".

Referring to FIG. 2, a training device 200 according to an embodiment of the present disclosure includes a database 210, a communicator 230, a processing unit 250, and an output unit 270. According to an embodiment, the training device 200 according to the present disclosure may be implemented in a form in which the communicator 230 and the output unit 270 are included in the processing unit 250 and thus may be implemented to be substantially operable only with the database 210 and the processing unit 250.

The database 210 stores various types of data needed for the training device 200 to operate. As an example, the database 210 stores a program for executing a training algorithm for improving a cognitive function while communicating with the management server 20, and may perform a function of temporarily or semi-permanently storing data processed by the processing unit 250.

In the case where the training device 200 is installed on a user terminal, the communicator 230 performs processing for communicating with the management server 20. In detail, the communicator 230 may process an authentication procedure needed to execute the training device 200 while communicating with the management server 20. The authentication procedure may be performed by a prescription code issued by a doctor, and the description thereof is given below. In the case where the training device 200 is implemented in the management server 20, the communicator 230 may be omitted.

The processing unit 250 processes data received by the communicator 230 and data to be transmitted, and performs a function of providing and controlling a screen that is output by the training device 200 to a display of the user terminal. The processing unit 250 may refer to a data processing device having a structured circuit to perform a function expressed by a code or an instruction included in a program. For example, the processing unit 250 may be at least one of a microprocessor, a processor core, a multiprocessor, an application-specific integrated circuit (ASIC), and a field programmable gate array (FPGA). As described above, in the case where the training device 200 is not implemented as a physical device but is implemented only as a logical device, the processing unit 250 may be implemented in the form of a virtual machine that operates by imitating operation characteristics of at least one of a microprocessor, a processor core, a multiprocessor, an ASIC, and an FPGA.

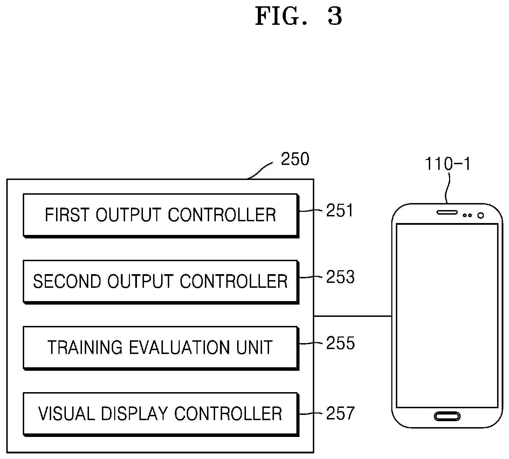

As an embodiment, the processing unit 250 may perform a function of combining data received by the communicator 230 with information stored in the database 210 and processing the same, or issuing a command so that the communicator 230 and the output unit 270 operate appropriately to implement a method according to the present disclosure. The function performed by the processing unit 250 is not limited to a particular function, and although the processing unit 250 is illustrated as a single module in FIG. 2, the processing unit 250 may be subdivided into a plurality of modules according to a process of the processing unit 250. The processing unit 250 including the subdivided modules is described below with reference to FIG. 3.

The output unit 270 performs a function of calculating and outputting various types of data by receiving the command from the processing unit 250. The output unit 270 performs a function of calculating and outputting various types of data by receiving the command from the processing unit 250 and according to an embodiment, may be implemented in a form included in the processing unit 250 to be omitted.

FIG. 3 is a block diagram illustrating an example of a processing unit subdivided by function.

FIG. 3 illustrates that a user terminal 110-1 is one of n user terminals included in the user terminal group 110 described with reference to FIG. 1, and the user terminal 110-1 refers to an electronic device including a display device that visually outputs a result of processing data by the user terminal 110-1, a speaker that audibly outputs the result of processing data, and an input device that receives an input by a user.

Referring to FIG. 3, the user terminal 110-1 is illustrated in the form of a smartphone, but the user terminal 110-1 when the present disclosure is implemented is not limited to the smartphone, and may include all of an apparatus installed at a fixed location such as a general personal computer (PC) and a portable terminal such as a tablet personal computer (PC) or a netbook. Accordingly, the training device 200 may be driven in a form included in another hardware device such as a microprocessor or a general-purpose computer system. In other words, the training device 200 may be intactly mounted on the user terminal 110-1 or may be implemented in a form that is connected to the user terminal 110-1 through a wired or wireless network to control various types of data that are output to a display and a speaker of the user terminal 110-1.

FIG. 3 illustrates that only a processing unit 250 is highlighted in the training device 200, and referring to FIG. 3, the processing unit 250 includes a first output controller 251, a second output controller 253, a training evaluation unit 255, and a visual display controller 257. FIG. 3 illustrates that the processing unit 250 and sub-modules of the processing unit 250 are only components for highlighting only characteristics of an embodiment of the present disclosure. Accordingly, according to a different embodiment from the embodiment illustrated in FIG. 3, those skilled in the art may understand that other general-purpose components may be further included in addition to the components illustrated in FIG. 3.

The first output controller 251 controls a first training algorithm to be output to a user terminal in response to an input into the user terminal. Here, the input into the user terminal indicates that a user gives a particular input into the user terminal through an input device, and may include not only inputting vowels and consonants to construct a sentence, but also a touch input onto a touch pad of the user terminal, a voice input into a speaker of the user terminal, a shake input such as holding and shaking the user terminal, and the like.

The user may perform training for improving cognitive impairment symptoms by checking a training algorithm that is output from the user terminal and giving an input corresponding to the training algorithm, and the first output controller 251 may control the first training algorithm to be output to the user terminal in the case where an appropriate input by the user is detected. Here, the appropriate input by the user may be authentication information for enabling the first training algorithm to be output. For example, the user may execute an app installed on the user terminal 110-1 and log in, and in the case where the user is not authorized to use the app, may perform a sign-up procedure through sign-in.

As an embodiment, the user may enable the first training algorithm to be output by inputting a prescription code received from a doctor in addition to a normal login procedure. The first output controller 251 may verify whether or not the input prescription code is a valid code, through communication with an electronic medical record (EMR) server, and may control the first training algorithm to be output in the case where the prescription code is valid. The prescription code may be eight digits, but is not limited thereto. According to an embodiment, the first output controller 251 may also include, in login information, even information authenticated by the prescription code and process the same.

In the present embodiment, the prescription code refers to a code issued by a family doctor of the user, and according to an embodiment, may be a time-limited code that loses validity thereof after a certain point in time. For example, the user may enable the first training algorithm to be output by receiving an issue of a prescription code that is valid only for a month in May, running an app installed on a terminal, and then inputting the prescription code. At this time, in the case where the user inputs the same-day code issued on May 1 on June 1, the first training algorithm may not be output, and here, a validity period of the prescription code may be set in advance to a mathematically, empirically, or experimentally appropriate value.

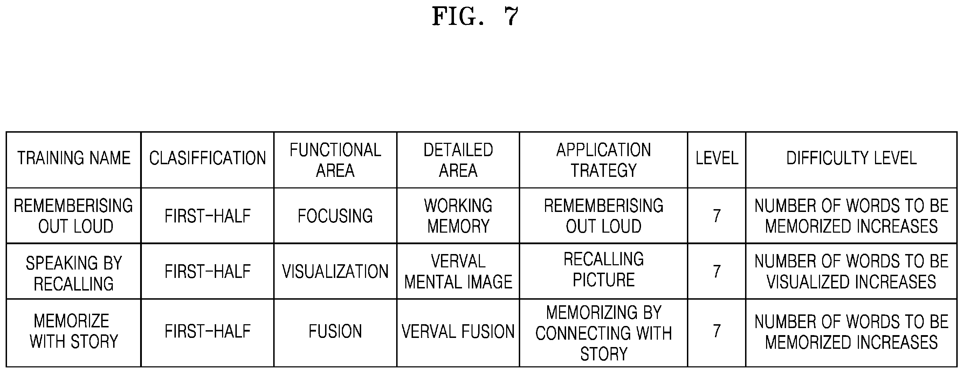

In the present disclosure, the first training algorithm refers to a regular training algorithm that is first performed to improve cognitive impairment symptoms of the user and is distinguished from a second training algorithm described below. The first training algorithm may include a plurality of detailed training algorithms, and may be treated as a kind of algorithm set due to characteristics including the plurality of detailed training algorithms. A detailed description of the first training algorithm is given with reference to FIG. 7.

In addition, the prescription code may be a combination of different alphabets and numbers for each detailed training algorithm included in the first training algorithm. For example, in the case where training A and training B are included as detailed training algorithms of the first training algorithm, a candidate group of prescription codes corresponding to the training A may be a₁, a₂, a₃, a₄, and a candidate group of prescription codes corresponding to the training B may be b₁, b₂, b₃, and b₄. In other words, at least two prescription codes may be matched to one training and stored. Here, a₁, b₃, or the like is represented as representative letters, and may actually be a code including a combination of a plurality of alphabets and numbers.

In the above example, when a one-month time-limited characteristic is additionally applied to the prescription code, in the case where a prescription code that the user needs to input to receive the training A in March (a prescription code issued by a doctor) is a₁, a prescription code that the user needs to input to receive the training A in April may be any one of a₂, a₃, and a₄. In addition, in the case where the user runs the app for a series of training and input the prescription code as a₁, a₂, b₃, and a₄, a server stores a training history of the user as a serial code, and the serial code is treated as history data regarding training experienced by the user and at the same time as metadata regarding the user, so that the doctor or the second output controller 253 may intuitively check the training history of the user and may use the same as base data for devising an effective treatment plan.

In the case where the first training algorithm is terminated, the second output controller 253 may control a second training algorithm to be output to the user terminal 110-1. Similar to the first training algorithm, the second training algorithm may also include a plurality of detailed training algorithms, and may be treated as a kind of algorithm set due to characteristics including the plurality of detailed training algorithms. The second output controller 253 may receive history information regarding the first output controller 251 executing and terminating the first training algorithm, and in the case where valid history information is present, may control the second training algorithm to be output to the user terminal 110-1. The detailed training algorithms included in the second training algorithm are different from the detailed training algorithms included in the first training algorithm, and in the case where primary training is performed on cognitive impairment symptoms of the user by the first training algorithm, the second training algorithm includes an algorithm for significantly amplifying an effect of the primary training. In other words, the second training algorithm refers to a training algorithm presupposing that the first training algorithm is preemptively performed.

As an embodiment, the second output controller 253 may control the second training algorithm to be output from the user terminal 110-1 after a certain time elapses after the first training algorithm is terminated. An effect of improving cognitive impairment symptoms, according to the present disclosure, may be expressed while the first training algorithm and the second training algorithm are sequentially and repeatedly performed, and in particular, to maximize a training effect of the second training algorithm, the user may perform training according to the first training algorithm and then rest the brain for a certain period of time. To maximize the training effect as described above, the second output controller 253 may control the second training algorithm to be output to the user terminal 110-1 only after a certain time elapses, and while a certain time does not elapse, may control the second training algorithm not to be output even in the case where the first training algorithm is terminated. Here, the certain time may be one hour, and according to an embodiment, a time shorter or longer than one hour may also be set.

In the present disclosure, the number of detailed training algorithms included in the first training algorithm may be a fixed first number. For example, the first number may be three, but is not limited thereto.

In addition, in the present disclosure, the number of detailed training algorithms included in the second training algorithm may be a third number selected from a fixed second number according to a preset condition. For example, the second number may be nine and the third number may be four, but are not limited thereto. The preset condition set in the second output controller 253 may vary.

In addition, in the present disclosure, the criterion for selecting the third number may be a result of evaluation of an input from the user. In the case where a detailed training algorithm, which is evaluated as vulnerable while the user performs training, is present, the detailed training algorithm may be essentially included in training included in the third number. The second output controller 253 may interwork with the training evaluation unit 255 to check strength and weaknesses of the user and induce training to proceed in a manner that compensates for the weaknesses. In other words, all of the first training algorithm is essentially performed, but only some of the detailed training algorithms included in the second training algorithm may be performed, and a criterion for selecting some thereof may be a training achievement level of the user.

Training for improving cognitive impairment symptoms, which is performed according to the present disclosure, may be performed on a daily basis. In other words, the first training algorithm and the second training algorithm may need to be sequentially performed on the same date to be effectively processed. For example, in the case where the first training algorithm is terminated at 23:30 on a particular date and a certain time needed to output the second training algorithm is set to one hour, the second output controller 253 may control the first output controller 251 to output the first training algorithm to the user terminal 110-1 as initial training of the date, not the second training algorithm, even in the case where the user executes the training app at 0:30 on the next day of the particular date after the first training algorithm is terminated. The same-day execution process of the first training algorithm and the second training algorithm is described below.

In the case where the second training algorithm is terminated, the training evaluation unit 255 may evaluate inputs by the user into the first training algorithm and the second training algorithm. Here, the inputs by the user into the first training algorithm and the second training algorithm may be training results for the training algorithms. When the first training algorithm or the second training algorithm is displayed through the user terminal 110-1, at least some of inputs that are input through an input device of the user terminal 110-1 may be a training result for the first training algorithm or the second training algorithm, and the training evaluation unit 255 collects training results and quantifies the collected training results into grades or scores indicating whether cognitive impairment symptoms of the user improve or worsen compared to the past.

The visual display controller 257 may control a first visual display to be output to the user terminal 110-1, on the basis of the result evaluated by the training evaluation unit 255. Here, the first visual display may be output through a display of the user terminal 110-1, and visual effects (a color, a shape, a pattern, a sparkle, and the like) of the first visual display may vary according to training fidelity and training performance for a training algorithm performed by the user.

As an example, the first visual display may be a flower of memory. **In** the present disclosure, the flower of memory is a representative example of a visual display that may be variously displayed as training for improving cognitive impairment symptoms of the user is repeated, and as the user more faithfully participates in training and the user performs the training better, has an improved appearance in proportion to the same.

The flower of memory may be changed in the appearance from a bud form to a full bloom form. In the present disclosure, the flower of memory may not only provide an aesthetic effect to the user, but also may represent a better visual effect according to the training fidelity or training performance of the user, and thus, may give training motivation to the user as the user participates in the training more diligently. In other words, the flower of memory improves sustainability of training.

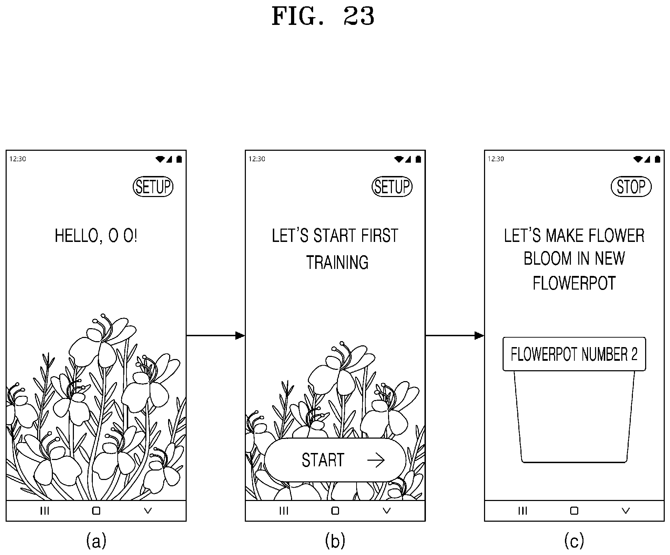

In addition, the visual display controller 257 may visually stimulate an area of the brain of the user in charge of a cognitive function and induce the cognitive impairment symptoms of the user to improve, by repeatedly and time-series showing a beautiful natural phenomenon such as the flower of memory. In addition, the user may feel a sense of achievement while visually checking an ever-increasing number of flowers of memory through the user terminal 110-1. A schematic description of the flower of memory is given below with reference to FIGS. 23 to 30.

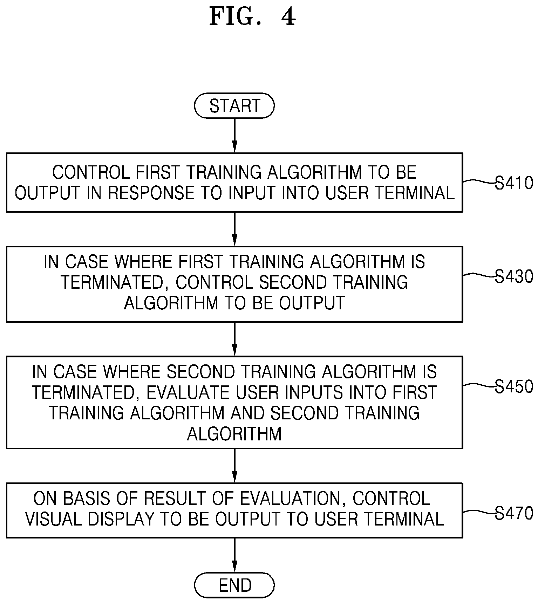

FIG. 4 is a flowchart illustrating an example of an operation process of submodules of a processing unit of FIG. 3.

A method according to FIG. 4 may be implemented by the first output controller 251, the second output controller 253, the training evaluation unit 255, and the visual display controller 257 described with reference to FIG. 3 and thus is described below with reference to FIG. 3, and the same description as the description of FIG. 3 is omitted.

The first output controller 251 may control a first training algorithm to be output in response to an input into a user terminal (S410).

In the case where the first training algorithm is terminated, the second output controller 253 may control a second training algorithm to be output (S430).

In the case where the second training algorithm is terminated, the training evaluation unit 255 may evaluate inputs by a user into the first training algorithm and the second training algorithm (S450).

The visual display controller 257 may control a visual display to be output to the user terminal on the basis of the result of the evaluation performed by the training evaluation unit 255 (S470).

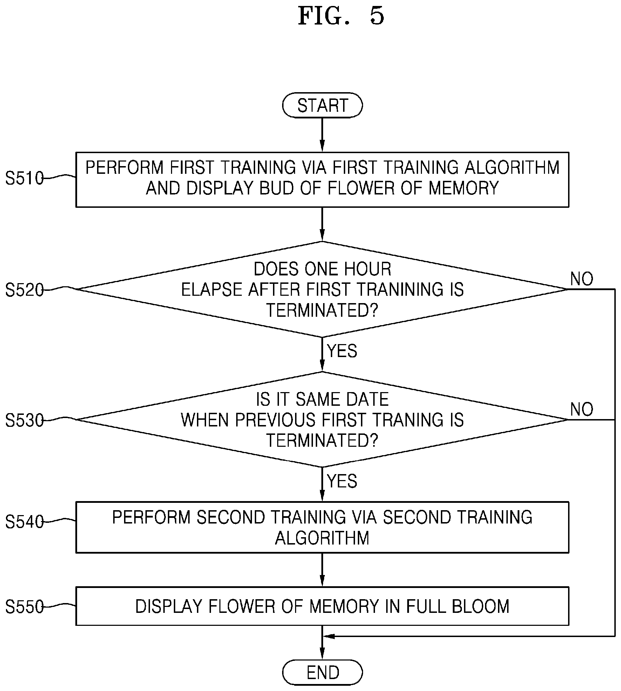

FIG. 5 is a flowchart illustrating an example of a process of a first training algorithm and a second training algorithm.

A method according to FIG. 5 may be implemented by the first output controller 251, the second output controller 253, the training evaluation unit 255, and the visual display controller 257 described with reference to FIG. 3 and thus is described below with reference to FIG. 3, and the same description as the description of FIG. 3 is omitted.

The first output controller 251 may perform first training by using a first training algorithm, according to an input by a user and may visually display a bud of a flower of memory (S510).

The second output controller 253 may check, through the first training algorithm, that the first training is terminated and in the case where an input by the user for starting a second training algorithm is given to a user terminal, determine whether or not an hour elapses after the first training is terminated (S520). Here, one hour is a certain time that may be changed through setting and thus may be a longer or shorter time value than one hour.

In the case where one hour elapses after the first training according to the first training algorithm is terminated, the second output controller 253 may determine whether or not a date on which second training according to the second training algorithm starts is the same as a date of the first training according to the first training algorithm immediately before (S530).

Only in the case where both the determinations in operations S520 and S530 are positive, the second output controller 253 allows the second training to be performed with the second training algorithm (S540), and after the second training is terminated, may display the flower of memory displayed in the form of the bud in operation S510 in the form of a full bloom (S550).

A schematic description of FIG. 5 is given below in the description of FIG. 23.

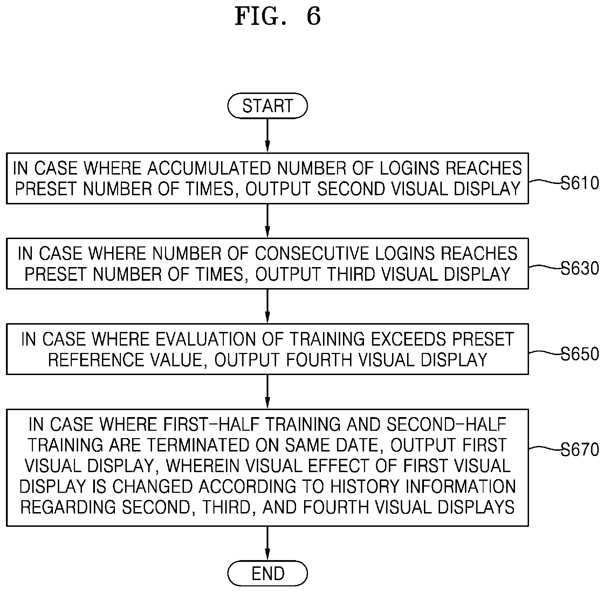

FIG. 6 is a flowchart illustrating an example of a process in which a visual effect of a first visual display is improved.

In the case where the accumulated number of logins of a user to a training app installed on the user terminal 110-1 reaches a preset number of times, the visual display controller 257 may control a second visual display to be output to the user terminal 110-1 (S610).

The visual display controller 257 may check the number of logins of the user to the training app installed on the user terminal 110-1 and in the case where the number of consecutive logins reaches a preset number of times, control a third visual display to be output to the user terminal 110-1 (S630).

In the case where evaluation of first training for a first training algorithm and second training for a second training algorithm exceeds a preset reference value, the visual display controller 257 may control a fourth visual display to be output to the user terminal 110-1 (S650).

In the case where the first training and the second training corresponding to first-half training and second-half training are terminated on the same date, the visual display controller 257 may output the first visual display, but may change a visual effect of the output first visual display by reflecting history information regarding the second visual display, the third visual display, and the fourth visual display in preceding operations S610, S630, and S650 (S670). For example, when the second visual display, the third visual display, and the fourth visual display are not output, in the case where the first visual display is a flower of memory in the form of a light purple full bloom, the first visual display having the visual effect that is changed after the second visual display, the third visual display, and the fourth visual display are output may be a flower of a memory in the form of a gold full bloom. A schematic description thereof is given below with reference to FIGS. 23 to 30.

FIG. 7 is a table illustrating an example of detailed training algorithms included in a first training algorithm.

Referring to FIG. 7, a total of three detailed training algorithms may be included in a first training algorithm, but is not limited thereto, and according to an embodiment, the number of detailed training algorithms included in the first training algorithm may be less than three or more than three.

The first training algorithm may include detailed training algorithms referred to as "memorizing out loud", "speaking by recalling", and "memorizing with a story", and each of the detailed training algorithms may be implemented at a total of seven difficulty levels.

As described above, a user may perform training on all of the detailed training algorithms included in the first training algorithm. As illustrated in FIG. 7, the detailed training algorithms included in the first training algorithm are each training for different functional areas, and include only training that may effectively stimulate brain areas responsible for a cognitive function of the user. In other words, the detailed training algorithms constituting the first training algorithm each perform a function of indirectly enhancing training efficiency of different detailed training algorithms and thus, different from detailed training algorithm included in a second training algorithm, are not randomly selected and are all performed.

Referring to FIG. 7, the detailed training algorithms included in the first training algorithm are training for functional areas of focusing, visualization, and fusion, and overlapping functional areas are not present.

In addition, as an embodiment, the detailed training algorithms included in the first training algorithm may be performed according to a preset order. For example, as illustrated in FIG. 7, as "memorizing out loud", "remembering by recalling", and "memorizing with a story" are sequentially performed, the first training algorithm enables effective stimulation to be provided to the brain areas responsible for the cognitive function of the user. The order in which the detailed training algorithms included in the first training algorithm are performed may be set in advance by all of the detailed training algorithms included in the first training algorithm being performed by the first output controller 251, and is a feature distinguished from the feature in which only some of the detailed training algorithms included in the second training algorithm are performed and thus the order thereof may not be set.

Referring to FIG. 7, the detailed training algorithms included in the first training algorithm are each determined to a total of seven difficulty levels, and as the difficulty level increases, the number of words that the user needs to remember increases.

In the case where first training according to the first training algorithm is terminated, as stimuli for focusing, visualization, and fusion directly related to a cognitive functional area of a person are sequentially or randomly provided to the user, the user may be in an easy state to perform the second training algorithm including relatively high-level training compared to the first training algorithm.

When the detailed training algorithms included in the first training algorithm are output through a user terminal, in the case where a difficulty level is a first level, the description of a training method may be provided in the form of a narration. After listening to the description of the training method, the user may effectively perform training corresponding to the detailed training algorithms. An example of the narration provided for each detailed training algorithm is as follows.

First, when the detailed training algorithm "memorizing out loud" is provided to the user at a first difficulty level, the first output controller 251 may provide the user with a narration such as "I will memorize words by speaking out loud in this training", "I can memorize much better if I speak what to memorize out loud", or "Shall we practice by memorizing out loud?".

In addition, when the detailed training algorithm "speaking by recalling" is provided to the user at a first difficulty level, the first output controller 251 may provide the user with a narration such as "I will freely speak what comes to mind after looking at a word in this training", "I remember much longer if I clearly recall what to memorize in my head", and "Shall we practice with speaking by recalling?".

Finally, when the detailed training algorithm "memorizing with a story" is provided to the user at a first difficulty level, the first output controller 251 may provide the user with a narration such as "I will memorize words by creating a story in this training", "I can easily remember if I make what to memorize into a story", and "Shall we practice by memorizing with a story?".

The difficulty level of the detailed training algorithms may change every day. As an example, in the case where the user who starts "memorizing out loud" at the first difficulty level shows a high level of achievement, "memorizing out loud" on the next day may be provided at a higher difficulty level than the first level.

As another example, in the case where the user who starts "memorizing with a story" at a second difficulty level shows a low level of achievement, "memorizing with a story" on the next day may be provided at a lower difficulty level than the second level.

The first output controller 251 may control a narration for each detailed training algorithm to be provided only once initially. The above control method is to minimize waste of time of the user by omitting a narration in the case where the user who experiences a detailed training algorithm at a second difficulty level is trained with a detailed training algorithm at a first difficulty level.

Evaluation of achievement of the detailed training algorithms included in the first training algorithm may be basically performed in a manner of quantifying that the user accurately performs a requested behavior within a preset response time in the case where a message requesting a particular behavior related to a cognitive ability is output by speech. In other words, as the response time is set to be shorter, a difficulty level of training more increases, and a difficulty level of training demanding many behaviors in the same response time is set to be higher. In the case where the first training according to the first training algorithm is terminated, the first output controller 251 transmits a result value collected in a training process to the training evaluation unit 255, and the training evaluation unit 255 separately stores criteria for a word-type response and a sentence-type response according to a training type or a response type of the user that is internally defined in a detailed training algorithm, and then quantifies and outputs evaluation of the training.

For example, when the user receives a message for the word-type response and tries to recall one word and respond, a response time (a waiting time) that may be considered to be a correct answer is set to five seconds, and when the user tries to recall ten words and respond, a response time that may be considered to be a correct answer is set to 28 seconds.

As another example, when the user receives a message for the sentence-type response and tries to freely visualizes or identify a main point, a response time that may be considered to be a correct answer may be set to 20 seconds, and when the user tries to reconstructs a sentence by fusing four words, a response time that may be considered to a correct answer may be set to 50 seconds. In other words, a length of a response time that may be considered to be a correct answer may be individually set according to a type to which the user responds and characteristics of a detailed training algorithm.

Seven difficulty levels are set for each detailed training algorithm, and the difficulty level may initially start at a first level and then increase or decrease by considering an achievement level of the user. Due to the characteristics in which the first training algorithm includes the detailed training algorithms that need to be necessarily performed, and thus, the difficulty level is not adjusted for each detailed training algorithm, and the difficulty level may be commonly adjusted according to a correct answer rate of a detailed training algorithm that has a lowest correct answer rate.

Meanwhile, difficulty levels of the detailed training algorithms included in the second training algorithm may be adjusted for each detailed training algorithm. For example, when the first training algorithm includes three detailed training algorithms, difficulty levels thereof may all be three levels, and when the second training algorithm includes four detailed training algorithms, difficulty levels thereof may be adjusted individually, such as three levels, five levels, one level, and four levels in order.

An average correct answer rate for recent three times needs to be 80 % or more to increase a difficulty level by one level. In addition, the average correct answer rate for the recent three times needs to be less than 50 % to lower the difficulty level by one level. In the case where the average correct answer rate for the recent three times is 50 % to 80 %, the difficulty level does not change, and in the case where the concept of the average correct answer rate for the recent three times may not be established because the user has no training history, a first difficulty level may be maintained until the recent three times of training are established.

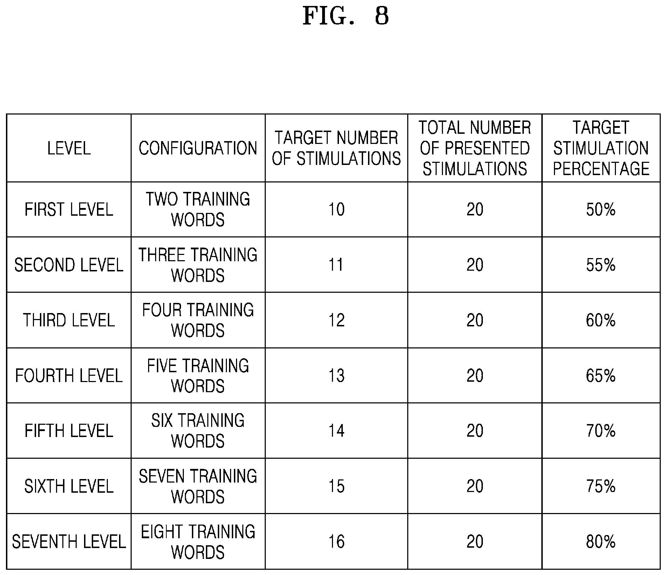

FIG. 8 is a table illustrating a criterion for changing a difficulty level of a detailed training algorithm corresponding to a focusing functional area in a first training algorithm.

In the present disclosure, with respect to a response of a user, by evaluating a correct answer rate for each detailed training algorithm and fine-tuning a difficulty level, cognitive impairment symptoms of the user may be effectively improved.

Referring to FIG. 8, at a first difficulty level, two training words are provided each time over a total of 20 times, and in the case where a correct answer of 50 % is confirmed, training is evaluated as successful. Meanwhile, at a seventh difficulty level, eight training words are provided each time over a total of 20 times, and in the case where a correct answer of 80 % is confirmed, training may be evaluated as successful. In the case where a target stimulation percentage is not reached, training is considered to fail and thus a difficulty level may be lowered when a next detailed training algorithm is performed.

As illustrated in FIG. 7, an example of a detailed training algorithm corresponding to the focusing functional area includes training "memorizing out loud". In the training "memorizing out loud", a process of speaking out loud trains to facilitate memory, and through the present training algorithm, the user is repeatedly trained to increase a working memory capacity by gradually increasing information to remember.

The first output controller 251 may control an output of "memorizing out loud" through the following process. First, the first output controller 251 may provide one word at a time for the user to memorize, and when providing the words, provide text and speech together, and provide only one word on one screen. Subsequently, the first output controller 251 induces the user to read the words to be memorized three times per one word, and in the process of reading along the words to be memorized, induces the user to receive three-dimensional stimulation of the focusing functional area by adding any one of loud, fast, and slow options.

The first output controller 251 provides feedback corresponding to a correct answer in the case where the user responds according to an instruction and controls feedback corresponding to an incorrect answer to be provided in the case where the user responds differently from the instruction or does not respond, and the feedback corresponding to the correct answer/the incorrect answer is set differently for each detailed training algorithm.

As a selective embodiment, the first output controller 251 may provide a high training effect by adding an interference word starting from a certain difficulty level. The interference word is similar to words memorized by the user, but it is not the same word. A non-response is considered a correct answer only when the user must not respond upon seeing the interference word on the screen.

The first output controller 251 may control the detailed training algorithm for the focusing functional area to be performed with words stored in the database 210. More than 224 words may be pre-stored in the database 210 by collectively considering the feature in which the difficulty level of "remembering out loud" includes eight levels, the feature in which the maximum exposure frequency of weekly training is seven times, and the feature in which a recommended use period of a training device for improving cognitive impairment symptoms is four weeks. In particular, considering an interference word for pre-stored words, words corresponding to n times of 224 words (n is a natural number greater than 1) may be stored in the database 210, and the stored words may be called by the first output controller 251.

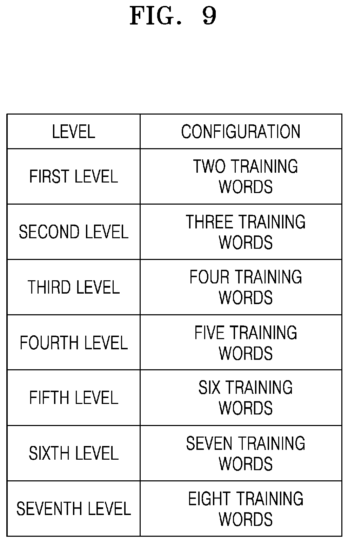

FIG. 9 is a table illustrating a criterion for changing a difficulty level of a detailed training algorithm corresponding to a visualization functional area in a first training algorithm.

Referring to FIG. 9, two training words are provided at a first difficulty level to allow a user to perform training corresponding to a visualization functional area, and training words increase to eight at a seventh difficulty level that is the highest difficulty level.

As illustrated in FIG. 7, an example of the detailed training algorithm corresponding to the focusing functional area includes training "speaking by recalling". The goal of the training "speaking by recalling" is to facilitate a memory by allowing a user to practice remembering by using experiences and imaginations.

The training "speaking by recalling" is training in which one word is provided to the user terminal 110-1 to allow the user to see the provided word for 3 seconds and think about what comes to mind, and in the case where the user first encounters the training, the first output controller 251 controls an example sentence to be output together with a user training description. For example, the first output controller 251 controls a guidance message "see a word and freely speak what comes to mind" to be output through a speaker of the user terminal 110-1 and collects speech of the user that is input after a certain waiting time (3 seconds) elapses.

The training evaluation unit 255 stores a scoring criterion of the training "speaking by recalling". As an example, in the case where the user responds within a time and at the same time, the user input includes at least five syllables, the user response may be evaluated as a correct response. A response form of the user is not standardized due to characteristics of the visualization functional area, and thus, the user response is determined as a concept of the correct response rather than a concept of a correct answer.

The first output controller 251 provides feedback corresponding to the correct response in the case where the user responds in the correct response, controls feedback corresponding to an incorrect response to be provided in the case where the user responds differently from an instruction or does not respond, and the feedback corresponding to the correct/incorrect response is set differently for each detailed training algorithm.

The first output controller 251 may control an example of the correct response to a corresponding word to be output to the user terminal 110-1 only once and instruct the user to read the example of the correct response within a certain time in the case where the user does not respond in the correct response, and control the training evaluation unit 255 to add a certain score in the case where the user reads along the example of the correct response within a certain time. For example, in the case where the user voluntarily responds in the correct response, two scores may be given, and in the case where the user reads along the provided correct response according to the instruction, one score may be given.

The first output controller 251 may control a detailed training algorithm for a visualization functional area to be performed with words stored in the database 210. More than 224 words may be pre-stored in in the database 210 by considering the feature in which the difficulty level of "speaking by recalling" includes eight levels, the feature in which the maximum exposure frequency of weekly training is seven times, and the feature in which a recommended use period of a training device for improving cognitive impairment symptoms is four weeks. In particular, considering the example of the correct response to the pre-stored words, words corresponding to n times of 224 words (n is a natural number greater than 1) are stored in the database 210, and the stored words may be called by the first output controller 251.

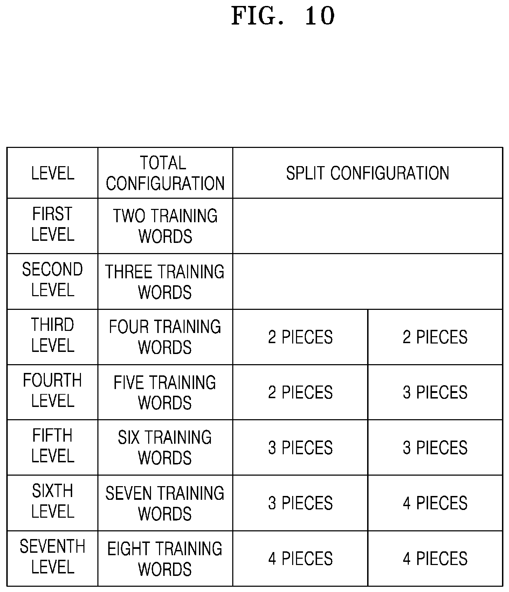

FIG. 10 is a table illustrating a criterion for changing a difficulty level of a detailed training algorithm corresponding to a fusion functional area in a first training algorithm.

Referring to FIG. 10, the first output controller 251 allows a user to perform training corresponding to a fusion functional area while two training words are provided at a first difficulty level, and training words increase to eight at a seventh difficulty level that is the highest difficulty level. In addition, the first output controller 251 controls training to be performed by dividing training words into two groups by using a split configuration starting from a third difficulty level.

As illustrated in FIG. 7, an example of the detailed training algorithm corresponding to the fusion functional area includes training "memorizing with a story". The goal of the training "memorizing with a story" is to facilitate a memory of a user by causing the user to repeat a process of weaving a story and remembering the same.

The training "memorizing with a story" refers to training that provides a certain number of words to the user terminal 110-1 and gives a story creation condition to induce the user to create a story that meets the provided words and the story creation condition. In the case where the user first encounters the training, the first output controller 251 may control a description of the training to be output to the user terminal 110-1.

The first output controller 251 may perform the training "memorizing with a story" through the following process. First, the first output controller 251 controls the number of training words corresponding to a difficulty level to be output to the user terminal 110-1, and allows the user to additionally check the story creation condition. Here, the story creation condition may be one of four emotional modifiers such as sad, happy, scary, and happy, and one may be randomly selected and provided to the user. The user are given a story creation time for 3 seconds, may create a story that satisfies both the training word and the story creation condition, and in the case where a speaking time arrives after the story creation time is terminated, may speak the created story for a set amount of time (e.g., 30 seconds).

The training evaluation unit 255 analyzes the story created by the user, comprehensively reviews whether or not all training words are included, whether or not the story creation condition is satisfied, and whether or not the story is terminated within a set time, and controls feedback on a correct response or an incorrect response to be provided to the user through the user terminal 110-1. For example, in the case where the user creates the story by putting only three of four training words, the first output controller 251 may receive feedback from the training evaluation unit 255 to notify the user of one missing word and induce the user to recreate a story. As described above, the training evaluation unit 255 may process so that a lower score is given in the case where the correct response does not come with one-time training.

As a selective embodiment, a time for which the user speaks the created story may be set differently by difficulty level. For example, the first output controller 251 causes the user to create a story about two training words at a first difficulty level and may give 30-second speaking time, but at a fourth difficulty level, causes the user to create a story about two training words and a story about three training words, and may give 20-second and 30-second speaking times, respectively, to allow sufficient stimulation to be provided a cognitive functional area of the user.

As another embodiment, after a speaking time of the user for the story is terminated, the first output controller 251 may ask again what a training word is and induce a response of the user. The user may receive sufficient stimulation in the cognitive functional area in a process of recalling again what training words are.

The training evaluation unit 255 stores a scoring criterion of the training "memorizing with a story". The first output controller 251 provides feedback corresponding to a correct response in the case where the user responds in the correct response and controls feedback corresponding to an incorrect response to be provided in the case where the user respond differently from an instruction or does not responds, and the feedback corresponding to the correct response/incorrect response is set differently by detailed training algorithm.

The first output controller 251 may control the detailed training algorithm for the focusing functional area to be performed with words stored in the database 210. More than 224 words may be pre-stored in the database 210 by considering the feature in which the difficulty level of "memorizing with a story" includes eight levels, the feature in which the maximum exposure frequency of weekly training is seven times, and the feature in which a recommended use period of a training device for improving of cognitive impairment symptoms is four weeks.

In the case where training "remembering out loud", "speaking by recalling", and "memorizing with a story" as detailed training algorithms included in the first training algorithm are all terminated, the first output controller 251 may provide the user through the user terminal 110-1 with feedback indicating that the first training algorithm is terminated and notify the visual display controller 257 that the first training algorithm is terminated to control a first visual display to be provided through the user terminal 110-1. As described above, the first visual display may be a flower of memory.

In addition, the first visual display provided by the visual display controller 257 may be an animation including at least two frames. For example, in the case where the first visual display is the flower of memory, after the first training algorithm is terminated, the visual display controller 257 may control an animation, which transitions from a first frame in which only a tree branch is drawn to a second frame in which a bud is formed on the tree branch, to be output through the user terminal 110-1. A visual display as described above is an image visually shaping that cognitive impairment symptoms of the user improve, and the user may see the flower of memory provided as the first visual display and feel confident that cognitive impairment symptoms are improved. A schematic description of the first visual display related to the flower of memory is given below with reference to FIGS. 23 to 30.

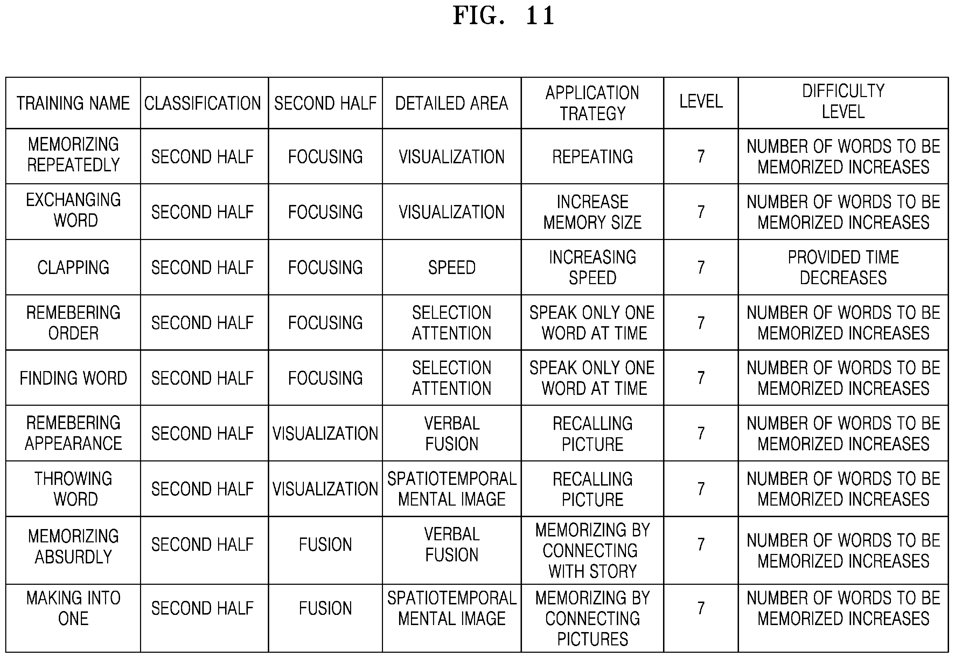

FIG. 11 is a table illustrating an example of detailed training algorithms included in a second training algorithm.

Referring to FIG. 11, a total of nine detailed training algorithms may be included in a second training algorithm. According to an embodiment, the number of detailed training algorithms included in the second training algorithm may be less than nine or more than nine.

The second training algorithm may include detailed training algorithms referred to as "memorizing repeatedly", "exchanging a word", "clapping", "remembering an order", "finding a word", "remembering appearance", "throw a word", "memorizing absurdly", and "making into one", and each of the detailed training algorithms may be implemented at a total of seven difficulty levels.

As described above, a user may perform training for some of the detailed training algorithms included in the second training algorithm, and a point in time at which the detailed training algorithms included in the second training algorithm are performed is after training is terminated with detailed training algorithms included in a first training algorithm. According to an embodiment, as described above, after the training is terminated with the detailed training algorithms included in the first training algorithm, the second training algorithm may start only after a certain time elapses.

As illustrated in FIG. 11, the detailed training algorithms included in the second training algorithm include only training that may effectively stimulate brain areas responsible for a cognitive function of the user. Referring to FIG. 11, the detailed training algorithms included in the second training algorithm are common to the detailed training algorithms included in the first training algorithm in that the detailed training algorithms are training in functional areas of focusing, visualization, and fusion, but are different from the detailed training algorithms included in the first training algorithm in that some overlapping functional areas are present. For example, in FIG. 11, five detailed training algorithms are present for the focusing functional area, two detailed training algorithms are present for the visualization functional area, and two detailed training algorithms are present for the fusion functional area.

Referring to FIG. 11, the detailed training algorithms included in the second training algorithm each have determined a total of seven difficulty levels, and as the difficulty level increases, the number of words that the user needs to remember increases.

In the case where first training according to the first training algorithm is terminated, as stimuli for fusing, visualization, and fusion directly related to a cognitive functional area of a person are sequentially or randomly provided to the user, the user may be in an easy state to perform the second training algorithm including relatively high-level training compared to the first training algorithm. In the case where detecting that the first training algorithm is terminated, the second output controller 253 may designate some of the detailed training algorithms included in the second training algorithm and control the same to be output to the user terminal 110-1.

When the detailed training algorithms included in the second training algorithm are output through the user terminal 110-1, in the case where a difficulty level is at a first level, a description of a training method may be provided in the form of a narration. After listening to the description of the training method, the user may effectively perform training corresponding to the detailed training algorithms. An example of the narration provided for each detailed training algorithm is as follows.

First, when the detailed training algorithm "remembering repeatedly" is provided to the user at a first difficulty level, the second output controller 253 may provide the user with a narration such as "I will memorize words by speaking repeatedly in this training", "Speaking repeatedly multiple times is a good memory method", or "Shall we practice with memorizing repeatedly?".

In addition, when the detailed training algorithm "exchanging a word" is provided to the user at a first difficulty level, the second output controller 253 may provide the user with a narration such as "I will alternately speak words that fit a topic in this training", "If you increase the amount you can remember little by little, you can increase the range of your memory", and "Shall we practice with exchanging a word?".

In addition, when the detailed training algorithm "clapping" is provided to the user at a first difficulty level, the second output controller 253 may provide the user with a narration such as "I will clap only on words I memorized in this training", "If you increase the speed little by little, it can give healthy stimulation to the brain", and "Shall we practice with clapping?".

In addition, when the detailed training algorithm "remembering an order" is provided to the user at a first difficulty level, the second output controller 253 may provide the user with a narration such as "In this training, I will be a store owner and remember an order from a customer", "If you focus on one thing at a time, you will not miss important things", or "Shall we practice with remembering an order?".

In addition, when the detailed training algorithm "finding a word" is provided to the user at a first difficulty level, the second output controller 253 may provide the user with a narration such as "I will focus only on what to remember among various types of information in this training", "Focusing on just one important thing helps concentration and memory.", and "Shall we practice with finding a word?".

In addition, when the detailed training algorithm "throwing a word" is provided to the user at a first difficulty level, the second output controller 253 may provide the user with a narration such as "I will listen to an explanation and recall the appearance of an object in this training", "If I more vividly recall the appearance of an object, I can remember it better", or "Shall we practice by remembering the appearance?".

In addition, when the detailed training algorithm "remembering the appearance" is provided to the user at a first difficulty level, the second output controller 253 may provide the user with a narration such as "I will try to memorize words by throwing an object into the sea in this training", "If I more vividly recall a scene where the object is thrown, I can more clearly leave it in my memory", or "Shall we practice with throwing a word?".

In addition, when the detailed training algorithm "memorizing absurdly" is provided to the user at a first difficulty level, the second output controller 253 may provide the user with a narration such as "I will memorize words with an absurd story in this training", "The more absurd and strange story stays in your head longer", or "Shall we practice with memorizing absurdly?".

In addition, when the detailed training algorithm "making into one" is provided to the user at a first difficulty level, the second output controller 253 may provide the user with a narration such as "I will memorize two words by making them into one in this training", "Imagining making into one is unfamiliar but is an effective memory method", or "Shall we practice with making into one?".

The second output controller 253 may control a narration of each detailed training algorithm to be provided only once initially. The above control method is to minimize waste of time of the user by omitting the narration in the case where the user who experiences a detailed training algorithm at a second difficulty level is trained with a detailed training algorithm at a first difficulty level.

The difficulty levels of the detailed training algorithms included in the second training algorithm may be adjusted by detailed training algorithm. For example, when the first training algorithm includes three detailed training algorithms, difficulty levels thereof may all be three levels, and when the second training algorithm includes four detailed training algorithms, difficulty levels thereof may be adjusted individually, such as three levels, five levels, one level, and four levels in order.

An average correct answer rate for recent three times needs to be 80 % or more to increase a difficulty level by one level. In addition, the average correct answer rate for the recent three times needs to be less than 50 % to lower the difficulty level by one level. In the case where the average correct answer rate for the recent three times is 50 % to 80 %, the difficulty level does not change, and in the case where the concept of the average correct answer rate for the recent three times may not be established because the user has no training history, a first difficulty level may be maintained until the recent three times of training are established.

Hereinafter, the training method and difficulty level setting of the detailed training algorithms included in the second training algorithm are described in detail.

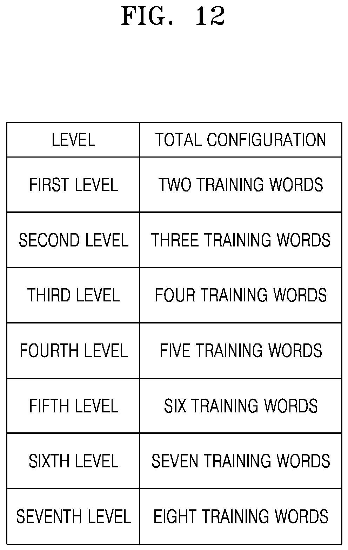

FIG. 12 is a table illustrating a criterion for changing a difficulty level of training "memorizing repeatedly" in a second training algorithm.

Referring to FIG. 12, the second output controller 253 allows a user to perform training "remembering repeatedly" while two training words are provided at a first difficulty level, and training words increase to eight at a seventh difficulty level that is the highest difficulty level. The goal of the training "memorizing repeatedly" is to induce the user to speak repeatedly to facilitate a memory of the user.

The second output controller 253 provides one word at a time for the user to memorize through the user terminal 110-1, and when providing the words, provides text and voice together, but may control only one word to be provided on one screen. The second output controller 253 may allow the user to repeatedly read along words to be memorized n times per one word and provide a response time of two seconds per one repetition. Here, n is a number randomly selected from 3 to 5.

In the case where the user reads along all of training words, the second output controller 253 may give a preset time (e.g., five seconds) and induce the user to speak all of training words learned at a current difficulty level in order. The user needs to recall and speak two training words sequentially at a first difficulty level, and needs to recall and speak eight training words sequentially at an eighth difficulty level to receive recognition as a correct response. As the number of training words increases, a time provided to the user may increase.

As a selective embodiment, a scoring criterion and a difficulty level change criterion of training "remembering repeatedly" stored in the training evaluation unit 255 may be different from each other. For example, an act of repeatedly speaking a word n times as guided while the user acquires a training word does not affect a change in a difficulty level and may only be scored, and the training evaluation unit 255 may determine whether to increase or decrease the difficulty level only with an achievement level of training in which the user sequentially speaks the training words in a recall stage after acquiring all the training words.

The second output controller 253 may control the training "remembering repeatedly" to be performed with words stored in the database 210. More than 224 words may be pre-stored in the database 210 by considering the feature in which the difficulty level of "remembering repeatedly" includes eight levels, the feature in which the maximum exposure frequency of weekly training is seven times, and the feature in which a recommended use period of a training device for improving cognitive impairment symptoms is four weeks.

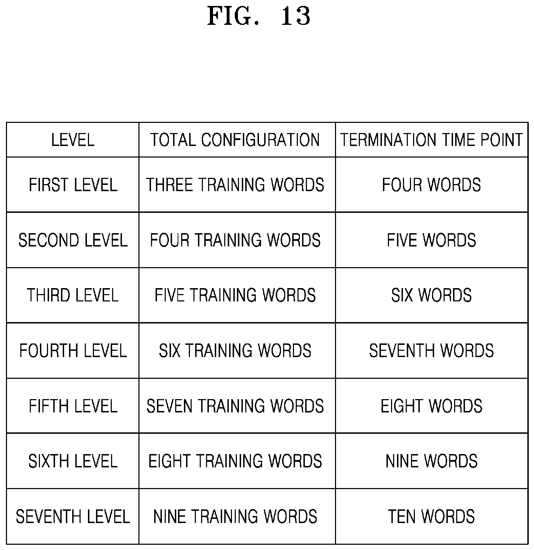

FIG. 13 is a table illustrating a criterion for changing a difficulty level of training "exchanging a word" in a second training algorithm.

Referring to FIG. 13, the second output controller 253 controls the training to be terminated at a point in time at which four training words are provided after three training words are provided at a first difficulty level and then to be terminated at a point in time at which ten training words are provided after nine training words are provided at a seventh difficulty level that is the highest difficulty level. **The** goal of the training "exchanging a word" is to increase a working memory capacity of a user by performing training for gradually increasing information to remember.

After providing three training words to the user through the user terminal 110-1, the second output controller 253 may induce the user to perform training for exchanging a word with an AI-based virtual training partner. In detail, the training "exchanging a word" refers to training in which, in the case where the user speaks a word "apple", AI responds with "apple" and "pear" and the user speaks again "apple", "pear", and "peach" and continuously speaks words one after another with words provided as training words.

The training evaluation unit 255 evaluates the result of training by the user ass a correct answer/incorrect answer on the basis of the number of training words set for each difficulty level and the number of words set at a termination time point. As an example, the case where the user speaks words one after another until the end with words set for each difficulty level may be processed as a correct answer, and any other cases may be processed as incorrect answers.

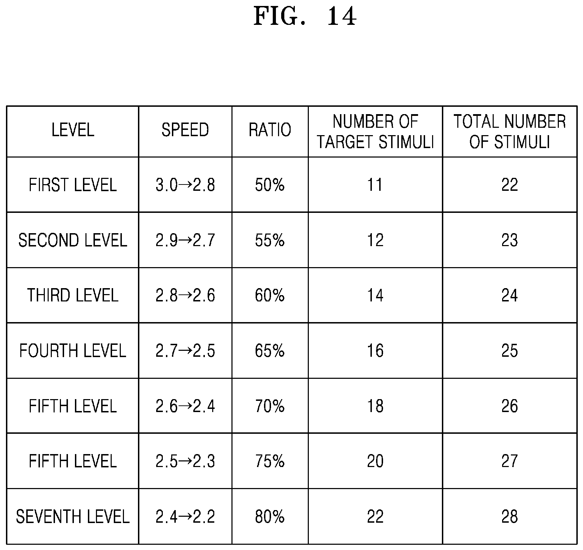

FIG. 14 is a table illustrating a criterion for changing a difficulty level of training "clapping" in a second training algorithm.

Referring to FIG. 14, at a first difficulty level, a clapping speed is reduced from 3.0 seconds to 2.8 seconds and a reference rate considered as a correct response is 50 %, but at a seventh difficulty level, the clapping speed is reduced from 2.4 seconds to 2.2 seconds and the reference rate considered as the correct response is significantly increased to 80 %. The goal of the training "clapping" is to improve a memory processing speed of a user by gradually shortening an execution time.

The second output controller 253 induces the user to memorize one word through the user terminal 110-1, to clap when the memorized word comes out, and not to clap when a word other than the memorized word comes out. The word other than the memorized word may become an interference word described above. At a seventh difficulty level, a word of a total of 28 times is output through the user terminal 110-1 without a speech guidance, and the user may be evaluated with respect to training by performing an act of checking a word within 2.2 seconds to 2.4 seconds and clapping or not clapping.

After a word is provided according to the total number of stimuli set for each difficulty level, the training evaluation unit 255 evaluates a correct response/incorrect response with respect to the result of training for the user on the basis of the number of claps. For example, the training evaluation unit 255 may evaluate as a correct response with respect to all stimuli in the case where the user accurately claps only for words memorized by the user.

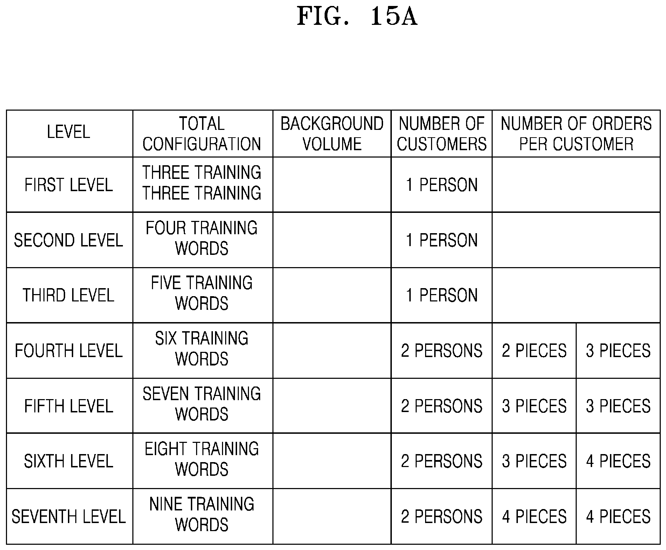

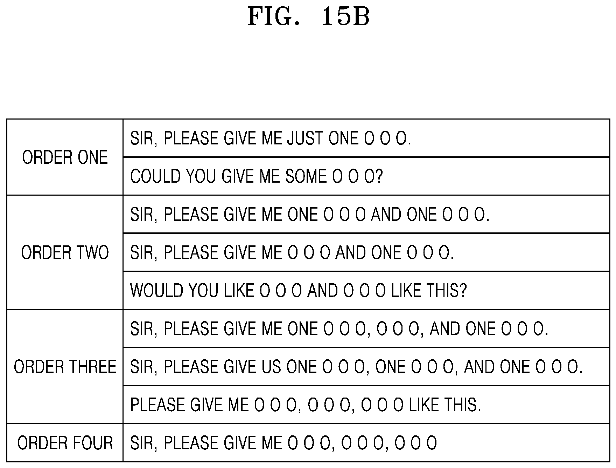

FIGS. 15A and 15B are tables illustrating criteria and example sentences for changing a difficulty level of training "remembering an order" in a second training algorithm.

Referring to FIG. 15A, at a first difficulty level, two training words are provided and the number of customers is one, but at a seventh difficulty level, eight training words are provided, the number of customers is two, and the number of orders per customer is four. The goal of the training "remembering an order" is to improve selective attention of a user having cognitive impairment symptoms through training that suppresses interference and focuses only on a goal.

The second output controller 253 may induce the user to memorize training words set for each difficulty level through the user terminal 110-1 and guide the user to listen to an order from at least one customer by using all of the training words set for each difficulty level. The user may receive an evaluation of a correct response/incorrect response to the present training through a procedure of listening to the order and checking once again a product ordered by the customer. Here, a voice of the customer may be implemented as a preset voice of AI.

Referring to FIG. 15B, various variations from when a customer orders one product to when the customer orders four products are described as example sentences. FIG. 15B is provided to the AI in the form of a script, and with reference to FIG. 15B, the AI may generate an order sentence and speak through the speaker of the user terminal 110-1.

The training evaluation unit 255 evaluates a correct response/incorrect response by determining how well the user designates a product that matches the order from the customer as a response. For example, in the case where at a seventh difficulty level, two customers each order four products and the user remembers seven products properly, the training evaluation unit 255 may finally evaluate the same as a correct response. According to an embodiment, to implement an actual situation, the second output controller 253 may also implement a kind of interference effect by causing background music (BGM) to be played back when a customer implemented as AI places an order for a product.

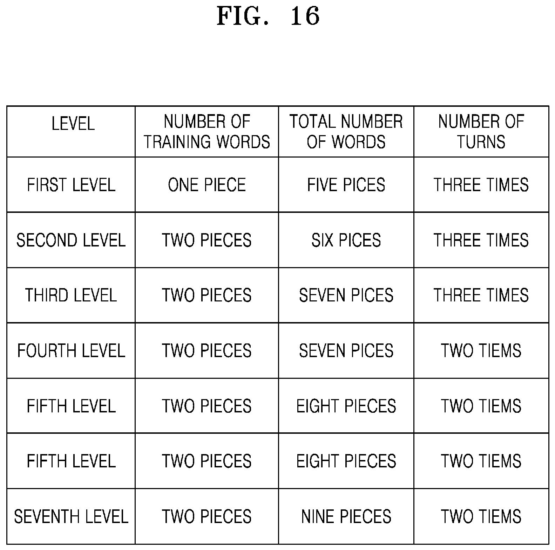

FIG. 16 is a table illustrating a criterion for changing a difficulty level of training "finding a word" in a second training algorithm.

Referring to FIG. 16, at a first difficulty level, a training word is one, total words are five, and the number of turns is three, but at a seventh difficulty level, training words are four, total words are nine, and the number of turns is two. The goal of the training "finding a word" is to improve selective attention of a user having cognitive impairment symptoms through training that suppresses interference and focuses only on a goal.

First, the second output controller 253 outputs a boundary through the user terminal 110-1 to induce the user to check the boundary. The boundary may be one randomly selected from among four categories of animal, transportation, occupation, and clothing, but another boundary may be added according to embodiments. Subsequently, the second output controller 253 may perform training to cause the user to speak a corresponding word only when the word corresponding to the boundary appears by changing and showing the word corresponding to the boundary and an interference word not corresponding to the boundary every two seconds. When a word is provided on the display of the user terminal 110-1, text and speech may be provided together.

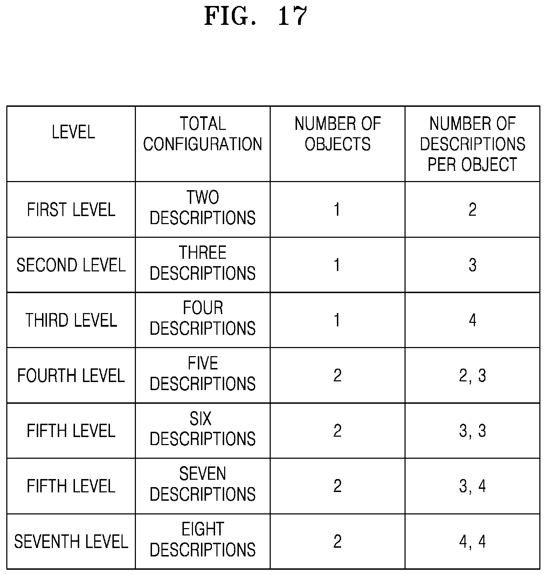

FIG. 17 is a table illustrating a criterion for changing a difficulty level of training "remembering the appearance" in a second training algorithm.

Referring to FIG. 17, a difference in the number of descriptions, a difference in the number of objects, and a difference in the number of descriptions per object are present between a first difficulty level and a seventh difficulty level. The training "remembering the appearance" is training that allows a user to create a clear mental image of information through a verbal description.

First, the second output controller 253 may control descriptions of an object to be output through the user terminal 110-1. The user may elicit a correct response/incorrect response to the training by listening to the descriptions of the object and responding to description elements. The description elements include seven elements such as a shape, a color, a pattern, a material, a number, a size, and a length. The second output controller 253 may describe a description of the object to the user in a manner of providing the description of the object in a sentence form, and instruct the user to imagine the appearance of the object for 5 seconds through the user terminal 110-1. Subsequently, the second output controller 253 may select one of the seven description elements from the user to request a response from the user, and the user may be trained by a method of responding thereto. As illustrated in FIG. 17, up to four description sentences for expressing each object are stored in the database 210.

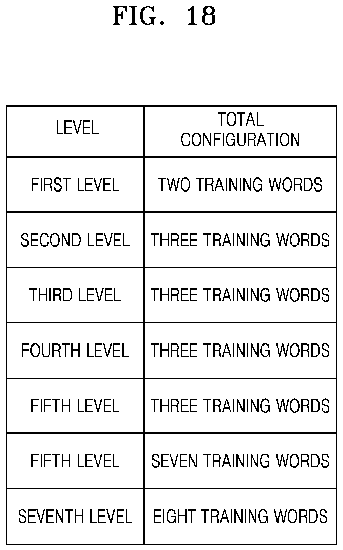

FIG. 18 is a table illustrating a criterion for changing a difficulty level of training "throwing a word" in a second training algorithm.

Referring to FIG. 18, a difference of six training words is present between a first difficulty level and a seventh difficulty level. The training "throwing a word" is a process to train a kinding process of unconsciously arranging information in a familiar space and remembering the same.

First, the second output controller 253 provides a word to be memorized by the user through the user terminal 110-1. When providing a word through the user terminal 110-1, the second output controller 253 may provide text and speech together. The second output controller 253 may control a guidance message to be output so that the user reads along a word to be memorized once. The second output controller 253 controls a description sentence of throwing a word to be memorized into the sea to be output regardless of whether or not the user responds, and instructs the user to sequentially speak training words thrown into the sea in the case where all the training words at each difficulty level are exhausted. The user recalls words by visualizing the image of the throwing the words into the sea, and as verified in statistical data, may recall the words with a higher memory than at other times. The training evaluation unit 255 may analyze a response of the user, determine how many training words are included, and evaluate the same with a score or grade.

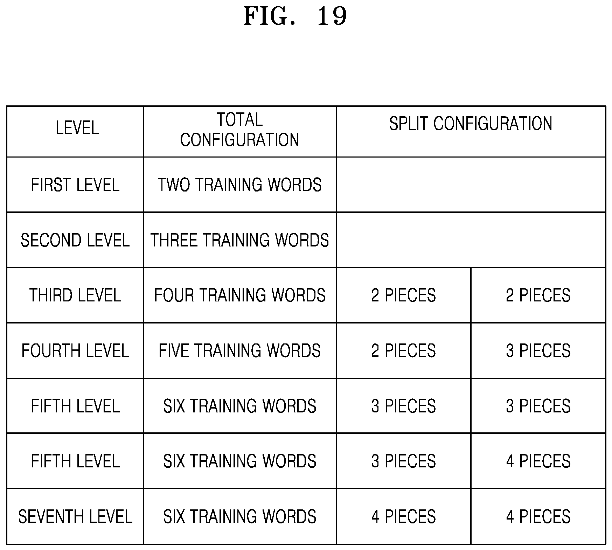

FIG. 19 is a table illustrating a criterion for changing a difficulty level of training "memorizing absurdly" in a second training algorithm.

Referring to FIG. 19, a difference of six training words is present between a first difficulty level and a seventh difficulty level, and a difference is also present in a split configuration. The split configuration indicates means that in the case were four training words are present, the same training is performed twice as when two training words are present, and a user is intensively stimulated in a corresponding cognitive functional area in a short period of time, and thus, efficiency of improving cognitive impairment symptoms may be increased. The training "memorizing absurdly" is training to train that a process of memorizing by weaving into a story facilitates a memory.

First, the second output controller 253 may provide, through the user terminal 110-1, a word (a training word) to be memorized by the user. When providing a word to the user, the second output controller 253 provides text and speech together, and may provide only one word on one screen. Subsequently, the second output controller 253 may provide a fusion sentence using both of provided two or more words. The user may read the fusion sentence and double-check two or more words. The second output controller 253 may instruct the user to recall and speak words memorized together for 5 seconds while providing only a portion of the fusion sentence as a clue. The training evaluation unit 255 may evaluate, as a correct response, the case where a word that the user recalls and speaks is a word included in the fusion sentence, evaluate any other cases as incorrect responses, and give scores.

The second output controller 253 may control the training "remembering absurdly" to be performed with words stored in the database 210. More than 224 words may be pre-stored in the database 210 by considering the feature in which a difficulty level of the training "memorizing absurdly" includes eight levels, the feature in which the maximum exposure frequency of weekly training is seven times, and the feature in which a recommended use period of a training device for improving cognitive impairment symptoms is four weeks. In addition, the training "memorizing absurdly" is performed by using a pre-stored fusion sentence, and thus, hundreds of fusion sentences for each of a second fusion to a fourth union are additionally stored in the database 210.

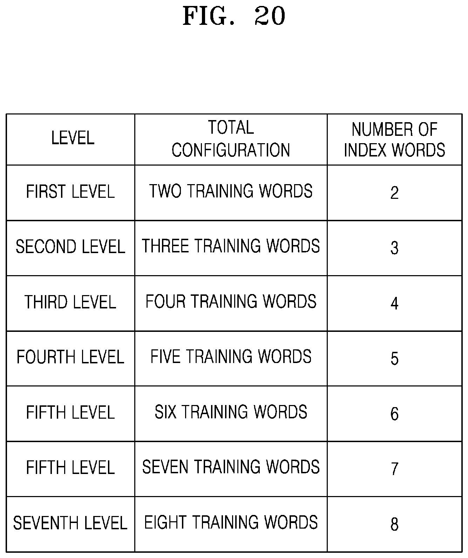

FIG. 20 is a table illustrating a criterion for changing a difficulty level of training "making into one" in a second training algorithm.

Referring to FIG. 20, not only a difference of six training words is present between a first difficulty level and a seventh difficulty, but also a difference of six index words is present. The index words are described below. The training "making into one" is training in the same fusion functional area as the training "memorizing absurdly" described above and thus is training to train a process of weaving into a story and remembering the same to facilitate a memory.

The second output controller 253 may provide one word at a time for the user to memorize. When providing a word through the user terminal 110-1, the second output controller 253 may provide text and speech together and may provide only one word on one screen. Subsequently, the second output controller 253 may provide one fusion image for each word to be memorized. The second output controller 253 may output a guidance message so that the user recalls an image for 5 seconds after checking all fusion images for respective words. Lastly, the second output controller 253 may provide a portion of the fusion image as a clue, instruct the user to speak words remembered together for 5 seconds, and count how many of provided training words come out, and control the training evaluation unit 255 to evaluate. The training evaluation unit 255 adds scores only in the case where all training words come out in a response of the user and process all other cases as score 0.

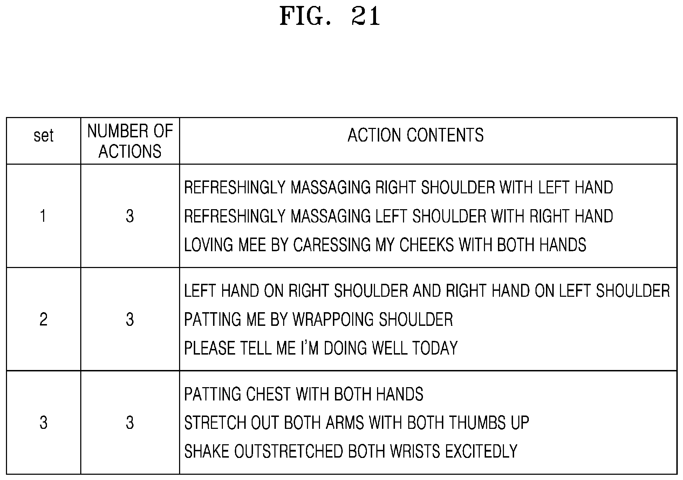

FIG. 21 is a table schematically illustrating a warming up algorithm that may be selectively performed before a first training algorithm is performed.

The warming up algorithm illustrated in FIG. 21 refers to a non-training algorithm that gives a certain stimulation to a cognitive functional area of a user by guiding the user to physically move before the first output controller 251 controls a first training algorithm to be output to a user terminal. As illustrated in FIG. 21, the user may make a condition of the user into the best condition before performing the first training algorithm, by moving the body and activate the brain according to a guidance provided by the first output controller 251.

The first output controller 251 controls the warming up algorithm illustrated in FIG. 21 to be provided alternately every day. For example, the first output controller 251 may control 1 set to be provided on Monday, 2 sets on Tuesday, 3 sets on Wednesday, and 1 set again on Thursday. A criterion for evaluating a performance achievement level of the warming up algorithm is not separately provided, and thus, the training evaluation unit 255 does not evaluate the warming up algorithm.

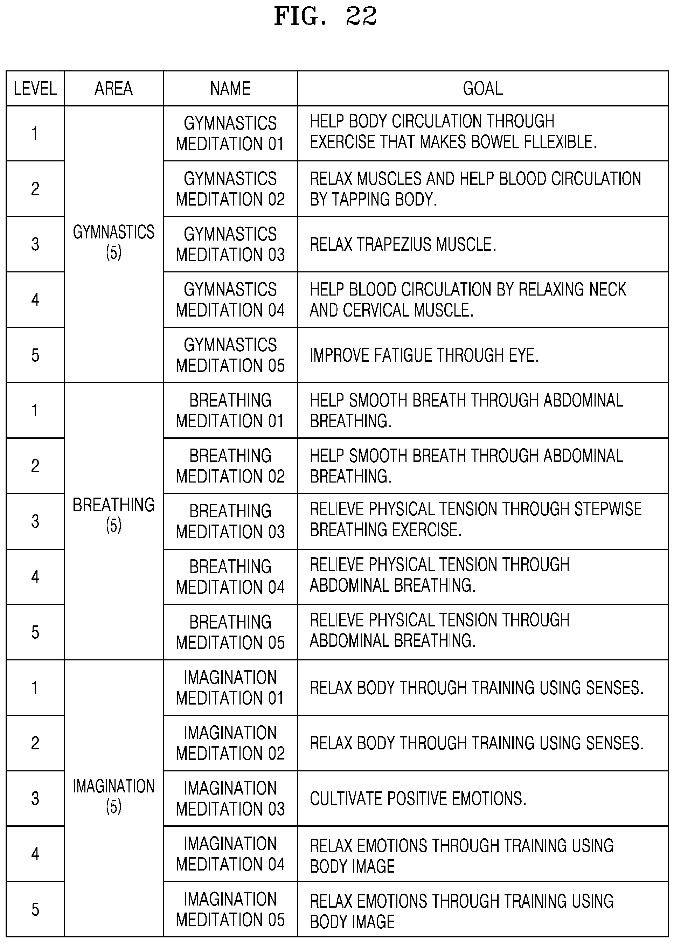

FIG. 22 is a table illustrating a meditation algorithm that may be selectively performed before a second training algorithm is performed.

Referring to FIG. 22, the meditation algorithm may be split into three areas such as gymnastics, breathing, and imagination. The number of detailed algorithms of the meditation algorithm stored in the database 210 is 60 or more, and FIG. 22 illustrates a result of extracting only some of the detailed algorithms. The meditation algorithm has a functional effect of relieving a tension state of a user before performing a second training algorithm that demands relatively higher concentration than a first training algorithm. The second output controller 253 may randomly select a preset number of detailed algorithms included in the meditation algorithm and provide the same to the user.

As a selective embodiment, in the case where some detailed training algorithms to be performed by the user in the present training are determined from among detailed training algorithms included in the second training algorithm, the second output controller 253 may control the meditation algorithm corresponding to the determined detailed training algorithms to be output. For example, in the case where the meditation algorithm corresponding to the training "throwing a word" is gymnastics meditation 5, the second output controller 253 may control the gymnastics meditation 5 to be necessarily included in a randomly selected detailed algorithm.

FIG. 23 is a view illustrating a first visual display that is output through a user terminal.

As described above with reference to FIG. 3, a first visual display controlled by the visual display controller 257 to be output to the user terminal 110-1 may be a flower of memory. The flower of memory refers to an image that shapes a flowerpot, a stem growing from the flowerpot, and a flower blooming from the stem.

(a) of FIG. 23 illustrates a screen output to the user terminal 110-1 when a user successfully logs in to a training app. In (a) of FIG. 23, a name of the user and a simple greeting are displayed together with flowers of memory.

(b) of FIG. 23 is a screen requesting a user input so that the user may start a first training algorithm. The user may give an input to a start button to cause (c) of FIG. 23 to be output.

(c) of FIG. 23 schematically illustrates the flowerpot in which the flower of memory is to grow. An indication "flowerpot number 2" indicates that the user already completes and archives "flowerpot number 1" by using the training app. The user may create and archive a total of four complete flowerpots while using the training app according to the present disclosure for four weeks. Seven stems may be grown in one flowerpot, and four completed flowerpots include a total of 28 stems. By archiving and then visually checking a flowerpot containing a flower of memory, the user may intuitively feel a sense of achievement of performing training for steadily improving cognitive impairment symptoms. An empty flowerpot is drawn in (c) of FIG. 23, but in the case where a previous training history is present, at least one stem may be displayed together with a flower of memory.

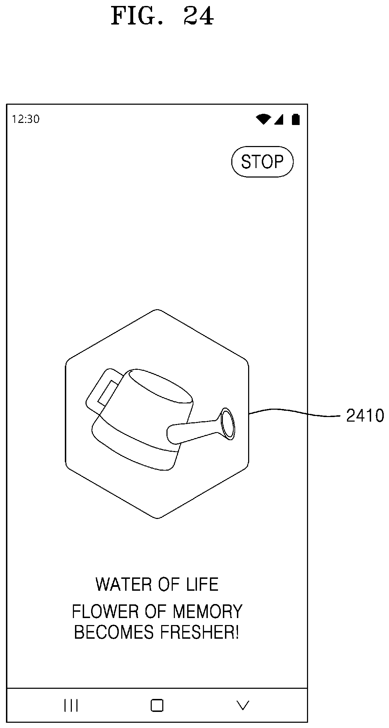

FIG. 24 is a view schematically illustrating an example of a badge that may be acquired by a user.

In more detail, in the case where a user logs into a training app through the user terminal 110-1, the visual display controller 257 may detect a login history before any one of a first training algorithm and a second training algorithm is executed and provide a reward on the basis of the detected login history. In the present disclosure, the reward may be provided to the user in the form of a badge or a flower of memory.

In FIG. 24, a water badge 2410 refers to a badge in which a watering can is engraved on a hexagonal support, and the water badge 2410 may be a reward provided in the case where the accumulated number of logins of the user reaches a certain number of times. Hereinafter, for convenience of description, the reward provided in the case where the accumulated number of logins reaches the certain number of times is referred to as a first reward. The first reward may be referred to as a second visual display following a flower of memory which is an example of a first visual display, in that the first reward is visually displayed on the user terminal 110-1.

A phrase for the water badge 2410 of FIG. 24 indicates that "water of life" is provided as a reward to make the flower of memory fresh because the accumulated number of logins of the user reaches the certain number of times, and the visual display controller 257 may control a visual effect of the first visual display, which is the flower of memory, to be changed on the basis of history information regarding the second visual display. For example, in the case where the water of life is applied, when a bud of the flower of memory transforms into a flower, the number of petals may be greater than usual. The user may feel a reward of training and obtain a sense of achievement through a visual change of the flower of memory.

As a selective embodiment, the certain number of times for providing the first reward to the user may be a constant temporal interval. For example, the visual display controller 257 may detect a fifth day login, a tenth day login, and a 15th day login to control the first reward to be provided.

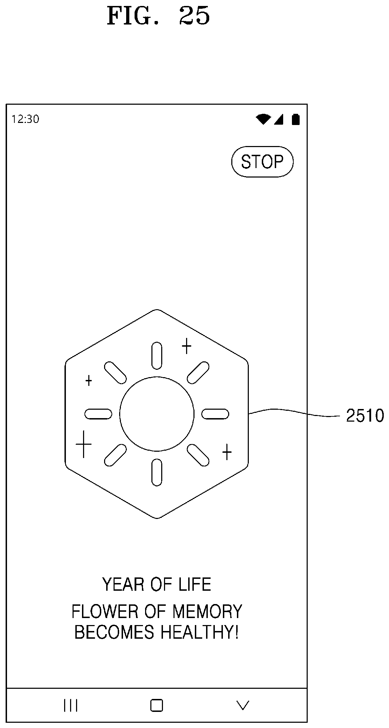

FIG. 25 is a view schematically illustrating another example of a badge that may be acquired by a user.

In FIG. 25, a solar badge 2510 refers to a badge in which the sun is engraved on a hexagonal support, and the sun in the sun badge 2510 is a result of shaping sunlight and sunshine. The solar badge 2510 may be a reward provided in the case where the number of consecutive logins of a user reaches a certain number of times. Hereinafter, for convenience of description, the reward provided in the case where the number of consecutive logins reaches a certain number of times is referred to as a second reward. Similar to the first reward, the second reward may be referred to as a third visual display following a flower of memory that is an example of a first visual display and a water badge that is an example of a second visual display, in that the second reward is visually displayed on the user terminal 110-1.

A phrase for the solar badge 2510 of FIG. 25 indicates that a "year of life" in which the flower of memory may become healthy because the number of consecutive logins of the user reaches a certain number of times is provided as a reward, and the visual display controller 257 may control a visual effect of the first visual display, which is the flower of memory, to be changed on the basis of history information regarding the third visual display. For example, in the case where the year of life is applied, a color of petals of the flower of memory may be changed to a darker and more intense color, and the user may feel a reward of training and obtain a sense of achievement through the visual change of the flower of memory.

As a selective embodiment, a certain number of times for providing the first reward to the user may be a date determined according to a certain rule. For example, the visual display controller 257 may detect three consecutive days of login, five consecutive days of login, ten consecutive days of login, and 20 consecutive days of login and control the second reward to be output.

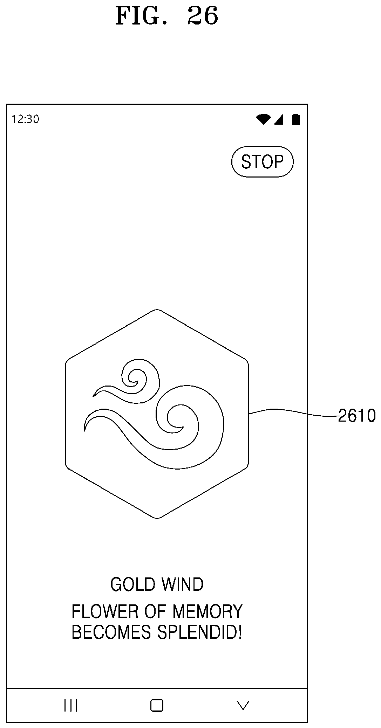

FIG. 26 is a view schematically illustrating another example of a badge that may be acquired by a user.

In FIG. 26, a gold wind badge 2610 indicates a badge in which a gold wind is engraved on a hexagonal support, and the gold wind of the gold wind badge 2610 is the result of shaping the wind. The gold wind badge 2610 may be a reward provided only in the case where an average correct answer rate, which is a result of evaluation by the training evaluation unit 255 in a second training algorithm performed by the second output controller 253, is 50 % or more. Hereinafter, for convenience of description, the reward provided in the case where the average correct answer rate is 50 % or more is referred to as a third reward. Similar to the first reward, the third reward may be referred to as a fourth visual display following the flower of memory that is an example of the first visual display, the water badge 2410 that is an example of the second visual display, and the solar badge 2510 that is an example of the third visual display, in that the third reward is visually displayed on the user terminal 110-1.

A phrase for the gold wind badge 2610 of FIG. 26 indicates that the "gold wind" in which the flower of memory may become colorful because the average correct answer rate of the user exceeds 50 % is provided as a reward, and the visual display controller 257 may control the visual effect of the first visual display, which is the flower of memory, to be changed on the bias of history information regarding the fourth visual display. For example, in the case where the gold wind is applied, the color of the petals of the flower of memory may be changed to a gold color, and the user may feel a reward of training and obtain a sense of achievement through the visual change of the flower of memory.

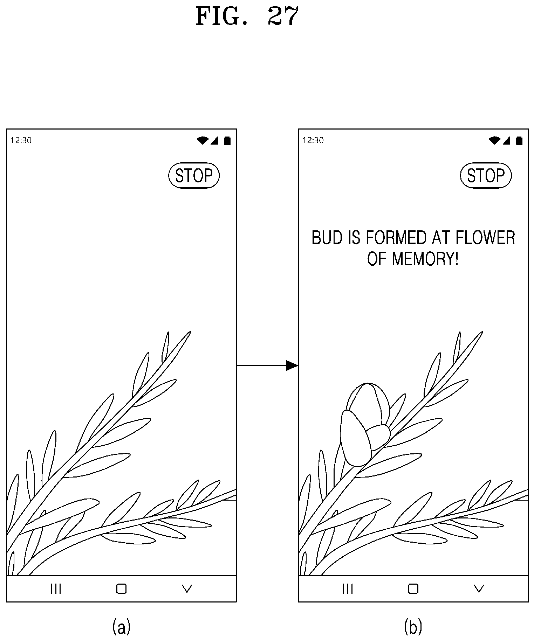

FIG. 27 is a view illustrating a bud of a flower of memory that is an example of a first visual display.

As described above, a flower of memory is an example of a first visual display, and in the case where a first training algorithm is normally terminated after a user logs in to be provided with a flowerpot, a bud of the flower of memory is formed on a stem, and in the case a second training algorithm is also normally terminated, as described above with reference to FIG. 3, the flower with the bud formed is changed to the form of a full bloom. FIG. 27 schematically illustrates a process in which the bud of the flower of memory is formed by the visual display controller 257 when the first training algorithm is normally terminated.

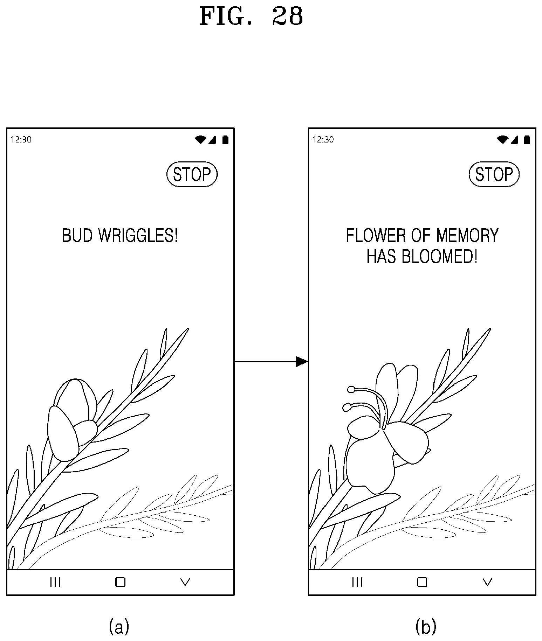

FIG. 28 is a view illustrating a flower of memory in full bloom, which is another example of a first visual display.

As described above, when a user normally performs training according to a first training algorithm and a bud of a flower of memory is formed, in the case where a second training algorithm is also normally terminated, the flower with a bud formed as in (a) of FIG. 28 is changed into a form of full bloom as in (b) of FIG. 28. The user may hope that cognitive impairment symptoms may be improved by sequentially viewing, through the user terminal 110-1, a state in which only a flowerpot is present, a state in which a bud of the flower of memory is formed on a stem grown in the flowerpot, and a state in which the flower of memory with the bud formed is in full bloom, feel a high sense of achievement and impression, and thus actively participate in training afterwards.

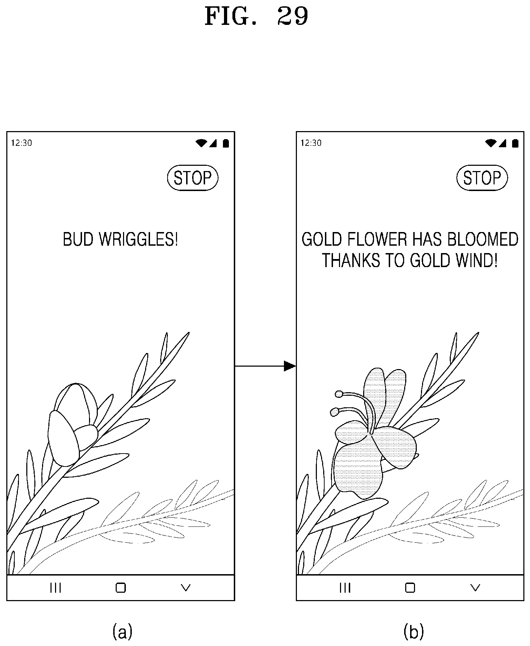

FIG. 29 is a view illustrating a flower of gold memory in full bloom, which is another example of a first visual display.

As described above, in the case where a user normally performs a first training algorithm and a second training algorithm and the training evaluation unit 255 determines that an average correct answer rate exceeds 50 %, a flower with a bud formed as in (a) of FIG. 29 is changed to a flower of gold memory in full bloom as in (b) of FIG. 29. The user may obtain a strong motivation for high achievement of training while viewing the flower of gold memory in full bloom through the user terminal 110-1. On the basis of history information in which the golden wind badge 2610 corresponding to the third reward is output as the fourth visual display, the visual display controller 257 may upgrade the flower of memory with a bud formed to a gold version and open the flower of memory, and may add a high-quality animation effect to a process of blooming the flower to provide a more dramatic effect to the user.

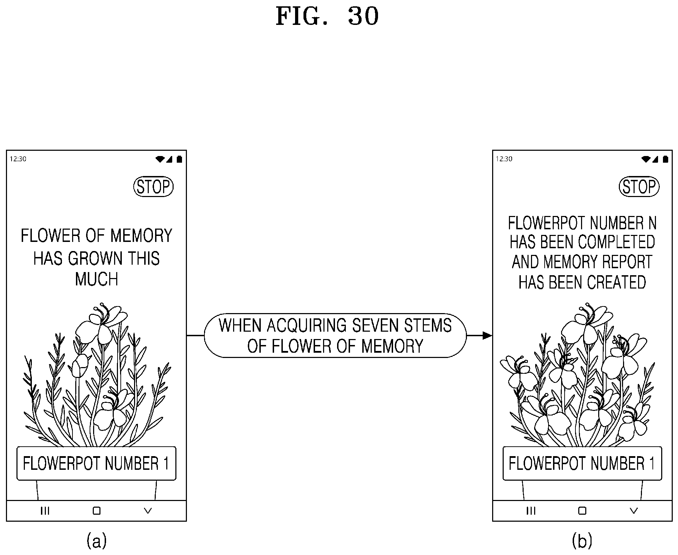

FIG. 30 is a view illustrating archiving of a flower of memory.

(a) of FIG. 30 illustrates that two flowers of memory are in full bloom, and one flower of memory has only a bud formed. In other words, a user normally completes training according to a first training algorithm and a second training algorithm for two days, and performs only training according to the first training algorithm for one day.

Meanwhile, (b) of FIG. 30 illustrates that seven flowers of memory in full bloom are hung on seven stems, and the user normally completes the training according to the first training algorithm and the second training algorithm without taking one day off for a week.

Each time new day starts, in the case where a stem grows from a flowerpot and training is not performed on the corresponding date, the training operates as a process in which a flower of memory is not be produced forever, and thus, both (a) and (b) of FIG. 30 illustrate a flowerpot in which the stem may no longer grow, and the flowerpot referred to as flowerpot number 1 is archived and remains permanently. The user may obtain aesthetic beauty and a sense of achievement while viewing the archived flowerpot. In the case where the flowerpot number 1 is archived, the flowerpot number 2 is newly provided on the next day, and thus, a flower of memory may be newly produced on the basis of the degree of participation of the user in training in the flowerpot number 2.

According to the present disclosure, a training algorithm for dramatically improving cognitive impairment symptoms of a patient having cognitive impairment symptoms may be provided in various forms.

The embodiments of the present disclosure described above may be implemented in the form of a computer program that may be executed through various components on a computer, and the computer program may be recorded on a computer-readable medium. Here, the medium may include magnetic media such as a hard disk, a floppy disk, and a magnetic tape, optical recording media such as CD-ROM and DVD, a magneto-optical medium such as a floptical disk, and a hardware device particularly configured to store and execute program instructions, such as ROM, RAM, and flash memory.

Meanwhile, the computer program may be particularly designed and configured for the present disclosure or may be known and available to those skilled in the art in the computer software field. Examples of the computer program may include not only machine language code, such as that produced by a compiler, but also high-level language code that may be executed by a computer by using an interpreter or the like.

Particular implementations described in the present disclosure are embodiments and do not limit the scope of the present disclosure in any way. For simplicity of the description, descriptions of existing electronic components, control systems, software, and other functional aspects of the systems may be omitted. Also, connections or connection members of lines between components illustrated in the drawing illustrate functional connections and/or physical or circuit connections, and may be represented in an actual apparatus as alternative or additional various functional connections, physical connections, or circuit connections. In addition, in the case where there are no particular mentions, such as "essential" and "significantly", the same may not be essential components for the application of the present disclosure.

In the description of the present disclosure (particularly, in claims), the use of the term "the" and similar indicative terms may correspond to both singular and plural forms. In addition, in the case where a range is described in the present disclosure, it is considered to include the disclosure that applies individual values belonging to the range (unless otherwise stated), and is the same as describing the respective individual values constituting the range in the detailed description of the disclosure. Finally, in the case where there is no explicit description or contrary description of the order of operations constituting a method according to the present disclosure, the operations may be performed in an appropriate order. The present disclosure is not necessarily limited to the order in which the operations are described. The use of all examples or example terms (e.g., and the like) in the present disclosure is merely intended to illustrate the present disclosure in detail and is not intended to limit the scope of the present disclosure by the examples or example terms, unless otherwise limited by claims. In addition, those skilled in the art may appreciate that various modifications, combinations, and changes may be configured according to design conditions and factors within the scope of the appended claims or equivalents thereof.

## Claims

1. A method of implementing an application for maintaining a cognitive function and improving a decline in the cognitive function, the method comprising:
controlling a first training algorithm to be output to a user terminal in response to an input into the user terminal;
in a case where the first training algorithm is terminated, controlling a second training algorithm to be output to the user terminal;
in a case where the second training algorithm is terminated, evaluating user inputs into the first training algorithm and the second training algorithm; and
on the basis of a result of the evaluation, controlling a first visual display to be output to the user terminal.

2. The method of claim 1, wherein the first training algorithm comprises a fixed first number of detailed training algorithms.

3. The method of claim 1, wherein the first training algorithm comprises a detailed training algorithm for a focusing functional area.

4. The method of claim 3, wherein the detailed training algorithm for the focusing functional area comprises an algorithm configured to receive and evaluate speech of a user using the user terminal.

5. The method of claim 3, wherein the detailed training algorithm for the focusing functional areas comprises:
providing a guidance message to the user through the user terminal; and
receiving the speech of the user having at least one of a pitch and a speed changed according to the guidance message, comparing the user speech with a stored value, and evaluating the user speech.

6. The method of claim 1, wherein the first training algorithm comprises a detailed training algorithm for a visualization functional area.

7. The method of claim 6, wherein the detailed training algorithm for the visualization functional area comprises an algorithm configured to receive the speech of the user using the user terminal and evaluate a number of syllables of a word included in the speech.

8. The method of claim 6, wherein the detailed training algorithm for the visualization functional area comprises:
providing a guidance message to the user through the user terminal; and
receiving the speech of the user for a certain period of time in response to the guidance message, detecting a number of syllables, comparing the detected number of syllables with a preset value, and evaluating the speech of the user.

9. The method of claim 1, wherein the first training algorithm comprises a detailed training algorithm for a fusion functional area.

10. The method of claim 9, wherein the detailed training algorithm for the fusion functional area comprises an algorithm configured to receive a story created by the user using the user terminal and determine whether or not the story meets a condition.

11. The method of claim 9, wherein the detailed training algorithm for the fusion functional area comprises:
providing a story creation condition to the user through the user terminal; and
in a case where the story created by the user is received, determining whether or not the story is a story corresponding to a number of words and emotional modifiers included in the story creation condition.

12. The method of claim 1, wherein the first training algorithm comprises at least two of the detailed training algorithms for the focusing, visualization, and fusion functional areas.

13. A computer program stored in a computer-readable storage device to execute the method of claim 1.

14. An apparatus for implementing an application for maintaining a cognitive function and improving a decline in the cognitive function, the apparatus comprising:
a first output controller configured to control a first training algorithm to be output to a user terminal in response to an input into the user terminal;
a second output controller configured to control a second training algorithm to be output to the user terminal in a case where the first training algorithm is terminated;
a training evaluation unit configured to evaluate user inputs into the first training algorithm and the second training algorithm in a case where the second training algorithm is terminated; and
a visual display controller configured to control a first visual display to be output to the user terminal on the basis of a result of the evaluation.
